# EUROPÄISCHE PATENTSCHRIFT

(11) **EP 1 912 994 B1**
(45) Veröffentlichungstag und Bekanntmachung des Hinweises auf die Patenterteilung: **12.03.2014**
(21) Anmeldenummer: 06776313.6
(22) Anmeldetag: 20.07.2006
(51) Int. Cl.: C07D 473/34, A61K 31/52, A61P 35/00

(54) **ADENINDERIVATE**
ADENINE DERIVATIVES
DERIVES D'ADENINE

(30) Priorität: 10.08.2005 DE 102005037733
(43) Veröffentlichungstag der Anmeldung: 23.04.2008
(73) Patentinhaber: Merck Patent GmbH, 64293 Darmstadt (DE)
(72) Erfinder: BUCHSTALLER, Hans-Peter, 64347 Griesheim (DE); EGGENWEILER, Hans-Michael, 64291 Darmstadt (DE); CEZANNE, Bertram, 64546 Moerfelden-Walldorf (DE); WOLF, Michael, 64297 Darmstadt (DE)
(86) Internationale Anmeldenummer: PCT/EP2006/007147
(87) Internationale Veröffentlichungsnummer: WO 2007/017069

(56) Entgegenhaltungen:
- WO-A-01/72779
- WO-A-2006/075095
- WO-A2-03/037860

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation von HSP90 eine Rolle spielt, ferner pharmazeutische Zusammensetzungen, die diese Verbindungen enthalten, sowie die Verwendung der Verbindungen zur Behandlung von Krankheiten, bei denen HSP90 eine Rolle spielt.

Die korrekte Faltung und Konformation von Proteinen in Zellen wird durch molekulare Chaperone gewährleistet und ist kritisch für die Regulation des Gleichgewichts zwischen Protein Synthese und Degradation. Chaperone sind wichtig für die Regulation vieler zentraler Funktionen von Zellen wie z.B. Zellproliferation und Apoptose (Jolly and Morimoto, 2000; Smith et al., 1998; Smith, 2001).

### Hitzeschock-Proteine (heat shock proteins, HSPs)

Die Zellen eines Gewebes reagieren auf äußerlichen Stress wie z.B: Hitze, Hypoxie, oxidativem Stress, oder Giftstoffen wie Schwermetallen oder Alkoholen mit der Aktivierung einer Reihe von Chaperonen, welche unter der Bezeichnung "heat shock proteins" (HSPs) bekannt sind.
Die Aktivierung von HSPs schützt die Zelle gegen Verletzungen, die durch solche Stressfaktoren ausgelöst werden, beschleunigt die Wiederherstellung des physiologischen Zustands und führt zu einem stresstoleranten Zustand der Zelle.
Neben diesem ursprünglich entdeckten durch HSPs vermittelten Schutzmechanismus bei äußerlichem Stress wurden im Laufe der Zeit weitere wichtige Chaperon-Funktionen für einzelne HSPs auch unter normalen stressfreien Bedingungen beschrieben. So regulieren verschiedene HSPs beispielsweise die korrekte Faltung, die intrazelluläre Lokalisierung und Funktion oder den geregelten Abbau einer Reihe biologisch wichtiger Proteine von Zellen.

HSPs bilden eine Genfamilie mit individuellen Genprodukten, deren Zellulärexpression, Funktion und Lokalisierung in verschiedenen Zellen sich unterscheidet. Die Benennung und Einteilung innerhalb der Familie erfolgt aufgrund ihres Molekulargewichts z.B. HSP27, HSP70, and HSP90.

Einigen menschlichen Krankheiten liegt eine falsche Proteinfaltung zugrunde (siehe Review z.B. Tytell et al., 2001; Smith et al., 1998). Die Entwicklung von Therapien, welche in den Mechanismus der Chaperon abhängigen Proteinfaltung eingreift, könnte daher in solchen Fällen nützlich sein. Beispielsweise führen bei der Alzheimer-Erkrankung, Prionenerkrankungen oder dem Huntington Syndrom falsch gefaltete Proteine zu einer Aggregation von Protein mit neurodegenerativem Verlauf. Durch falsche Proteinfaltung kann auch ein Verlust der Wildtyp-Funktion entstehen, der eine fehlregulierte molekulare und physiologische Funktion zur Folge haben kann.

HSPs wird auch eine grosse Bedeutung bei Tumorerkrankungen beigemessen. Es gibt z.B. Hinweise, dass die Expression bestimmter HSPs im Zusammenhang mit dem Stadium der Progression von Tumoren steht (Martin et al., 2000; Conroy et al., 1996; Kawanishi et al., 1999; Jameel et al., 1992; Hoang et al., 2000; Lebeau et al., 1991).

Die Tatsache, dass HSP90 bei mehreren zentralen onkogenen Signalwegen in der Zelle eine Rolle spielt und gewisse Naturstoffe mit krebshemmender Aktivität HSP90 targetieren, führte zu dem Konzept, dass eine Hemmung der Funktion von HSP90 bei der Behandlung von Tumorerkrankungen sinnvoll wäre.

Ein HSP90 Inhibitor, 17- Allylamino-17-demethoxygeldanamycin (17AAG), ein Derivat von Geldanamycin, befindet sich gegenwärtig in klinischer Prüfung.

### HSP90

HSP90 repräsentiert ungefähr 1-2% der gesamten zellulären Proteinmasse. Es liegt in der Zelle gewöhnlich als Dimer vor und ist mit einer Vielzahl von Proteinen, sogenannten Co-chaperonen assoziiert (siehe z.B. Pratt, 1997). HSP90 ist essentiell für die Vitalität von Zellen (Young et al., 2001) und spielt eine Schlüsselrolle in der Antwort auf zellulären Stress durch Interaktion mit vielen Proteinen, deren native Faltung durch äußerlichen Stress, wie z.B. Hitzeschock, verändert wurde, um die ursprüngliche Faltung wiederherzustellen oder die Aggregation der Proteine zu verhindern (Smith et al., 1998).
Es gibt auch Hinweise, dass HSP90 als Puffer gegen die Auswirkungen von Mutationen eine Bedeutung hat, vermutlich durch die Korrektur falscher Proteinfaltung, die durch die Mutation hervorgerufen wurde (Rutherford and Lindquist, 1998).
Darüber hinaus hat HSP90 auch eine regulatorische Bedeutung. Unter physiologischen Bedingungen spielt HSP90, zusammen mit seinem Homolog im Endoplasmatischen Retikulum, GRP94, eine Rolle im Zellhaushalt, um die Stabilität der Konformation und Reifung verschiedener "client" Schlüsselproteine zu gewährleisten. Diese können in drei Gruppen unterteilt werden: Rezeptoren für Steroidhormone, Ser/Thr or Tyrosinkinasen (z.B. ERBB2, RAF-1, CDK4 und LCK) und einer Sammlung unterschiedlicher Proteine wie z.B. mutiertes p53 oder die katalytische Untereinheit der Telomerase hTERT. Jedes dieser Proteine nimmt eine Schlüsselrolle in der Regulation physiologischer und biochemischer Prozesse von Zellen ein.
Die konservierte HSP90-Familie des Menschen besteht aus vier Genen, dem zytosolischen HSP90α, der induzierbaren HSP90β Isoform (Hickey et al., 1989), dem GRP94 im Endoplasmatischen Retikulum (Argon et al., 1999) und dem HSP75/TRAP1 in der mitochondrialen Matrix (Felts et al., 2000). Es wird angenommen, dass alle Mitglieder der Familie eine ähnliche Wirkweise haben, aber, je nach ihrer Lokalisierung in der Zelle, an unterschiedliche "client" Proteine binden. Beispielsweise ist ERBB2 ein spezifisches "client" Protein von GRP94 (Argon et al., 1999), während der Typ1 Rezeptor des Tumornekrosefaktors (TNFR1) oder das Retinoblastom Protein (Rb) als "clients" von TRAP1 nachgewiesen wurden (Song et al., 1995; Chen et al., 1996).
HSP90 ist an einer Reihe von komplexen Interaktionen mit einer grossen Zahl von "client" Proteinen und regulatorischen Proteinen beteiligt (Smith, 2001 ). Obwohl präzise molekulare Details noch nicht geklärt sind, haben biochemische Experimente und Untersuchungen mit Hilfe der Röntgenkristallographie in den letzten Jahren zunehmend Details der Chaperonfunktion von HSP90 entschlüsseln können (Prodromou et al., 1997; Stebbins et al., 1997). Danach ist HSP90 ein ATP-abhängiges molekulares Chaperon (Prodromou et al, 1997), wobei die Dimerisierung wichtig für die ATP Hydrolyse ist. Die Bindung von ATP resultiert in der Formation einer toroidalen Dimerstruktur, bei der die beiden N-terminalen Domainen in engem Kontakt zueinander kommen und einen "switch" in der Konformation bewirken. (Prodromou and Pearl, 2000).

### Bekannte HSP90 Inhibitoren

Die erste Klasse von HSP90 Inhibitoren, die entdeckt wurde, waren Benzochinon-Ansamycine mit den Verbindungen Herbimycin A und Geldanamycin. Ursprünglich wurde mit ihnen die Reversion des malignen Phänotyps bei Fibroblasten nachgewiesen, die durch Transformation mit dem v-Src Onkogen induziert worden war (Uehara et al., 1985).

Später wurde eine starke antitumorale Aktivität in vitro (Schulte et al., 1998) und in vivo in Tiermodellen gezeigt (Supko et al., 1995). Immunpräzipitation und Untersuchungen an Affinitätsmatrices zeigten dann, dass der Hauptwirkmechanismus von Geldanamycin eine Bindung an HSP90 involviert (Whitesell et al., 1994; Schulte and Neckers, 1998). Darüber hinaus wurde durch röntgenkristallographische Untersuchungen gezeigt, dass Geldanamycin um die ATP-Bindestelle kompetitiert und die intrinsische ATPase Aktivität von HSP90 hemmt (Prodromou et al., 1997; Panaretou et al., 1998). Dadurch wird die Entstehung des multimeren HSP90 Komplexes, mit seiner Eigenschaft als Chaperon für "client" Proteine zu fungieren, verhindert. Als Konsequenz werden "client" Proteine über den Ubiquitin-Proteasom-Weg abgebaut.
Das Geldanamycin Derivat 17- Allylamino-17-demethoxygeldanamycin (17AAG) zeigte unveränderte Eigenschaft bei der Hemmung von HSP90, der Degradation von "client" Proteinen und antitumoraler Aktivität in Zellkulturen und in Xenograft Tumormodellen (Schulte et al, 1998; Kelland et al, 1999), hatte aber eine deutlich geringere Leberzytotoxizität als Geldanamycin (Page et all 1997).17AAG wird gegenwärtig in Phasel/II klinischen Studien geprüft.

Radicicol, ein makrozyklisches Antibiotikum, zeigte ebenfalls Revision des v-Src und v-Ha-Ras induzierten malignen Phänotyps von Fibroblasten (Kwon et all 1992; Zhao et al, 1995). Radicicol degradiert eine Vielzahl von Signalproteinen als Konsequenz der HSP90 Hemmung (Schulte et al., 1998). Röntgenkristallographische Untersuchungen zeigten, dass Radicicol ebenfalls an die N-terminale Domäne von HSP90 bindet und die intrinsische ATPase Aktivität hemmt (Roe et al., 1998).

Antibiotika vom Coumarin Typ binden bekannterweise an die ATP Bindestelle des HSP90 Homologs DNA Gyrase in Bakterien. Das Coumarin, Novobiocin, bindet an das Carboxy-terminale Ende von HSP90, also an eine andere Stelle bei HSP90 als die Benzochinon-Ansamycine und Radicicol, welche an das N-terminale Ende von HSP90 binden.(Marcu et al., 2000b).

Die Hemmung von HSP90 durch Novobiocin resultiert in der Degradation einer großen Zahl von HSP90-abhängigen Signalproteinen (Marcu et al., 2000a).

Mit PU3, einem von Purinen abgeleiteten HSP90 Inhibitor konnte die Degradation von Signalproteinen z.B. ERBB2, gezeigt werden. PU3 verursacht Zellzyklus-Arrest und Differenzierung in Brustkrebs-Zelllinien (Chiosis et al., 2001).

### HSP90 als therapeutisches Target

Durch die Beteiligung von HSP90 an der Regulation einer großen Zahl von Signalwegen, die entscheidende Bedeutung am Phänotyp eines Tumors haben, und der Entdeckung, dass gewisse Naturstoffe ihren biologischen Effekt durch Hemmung der Aktivität von HSP90 ausüben, wird HSP90 gegenwärtig als neues Target für die Entwicklung eines Tumortherapeutikum geprüft (Neckers et al., 1999).

Der Hauptmechanismus der Wirkweise von Geldanamycin, 17AAG, und Radicicol beinhaltet die Hemmung der Bindung von ATP an die ATP-Bindestelle am N-terminalen Ende des Proteins und die daraus resultierende Hemmung der intrinsischen ATPase-Aktivität von HSP90 (siehe z.B. Prodromou et al., 1997; Stebbins et al., 1997; Panaretou et al., 1998). Die Hemmung der ATPase-Aktivität von HSP90 verhindert die Rekrutierung von Co-chaperonen und favorisiert die Bildung eines HSP90 Heterokomplexes, der "client" Proteine über den Ubiquitin-Proteasom-Weg der Degradation zuführt (siehe, z.B. Neckers et al., 1999; Kelland et al., 1999). Die Behandlung von Tumorzellen mit HSP90 Inhibitoren führt zur selektiven Degradation wichtiger Proteine mit fundamentaler Bedeutung für Prozesse wie Zellproliferation, Regulation des Zellzyklus und Apoptose. Diese Prozesse sind häufig in Tumoren dereguliert (siehe z.B. Hostein et al., 2001).
Eine attraktive Rationale für die Entwicklung eines Inhibitors von HSP90 ist, dass durch gleichzeitige Degradation mehrerer Proteine, die mit dem transformierten Phänotyp im Zusammenhang stehen, eine starke tumor-therapeutische Wirkung erreicht werden kann.

Im einzelnen betrifft die vorliegende Erfindung Verbindungen, die HSP90 hemmen, regulieren und/oder modulieren, Zusammensetzungen, die diese Verbindungen enthalten, sowie Verfahren zu ihrer Verwendung zur Behandlung von HSP90-bedingten Krankheiten, wie Tumorerkrankungen, virale Erkrankungen wie z.B. Hepatitis B (Waxman, 2002); Immunsuppression bei Transplantationen (Bijlmakers, 2000 and Yorgin, 2000); Entzündungsbedingte Erkrankungen (Bucci, 2000) wie Rheumatoide Arthritis, Asthma, Multiple Sklerose, Typ 1 Diabetes, Lupus Erythematodes, Psoriasis und Inflammatory Bowel Disease; Zystische Fibrose (Fuller, 2000); Erkrankungen im Zusammenhang mit Angiogenese (Hur, 2002 and Kurebayashi, 2001) wie z.B. diabetische Retinopathie, Hämangiome, Endometriose und Tumorangiogenese; infektiöse Erkrankungen; Autoimmunerkrankungen; Ischämie; Förderung der Nervenregeneration (Rosen et al., WO 02/09696; Degranco et al., WO 99/51223; Gold, US 6,210,974 B1); fibrogenetische Erkrankungen, wie z.B. Sklerodermie, Polymyositis, systemischer Lupus, Leberzirrhose, Keloidbildung, interstitielle Nephritis und pulmonare Fibrose (Strehlow, WO 02/02123).
Die Erfindung betrifft auch die Verwendung der erfindungsgemäßen Verbindungen zum Schutz normaler Zellen gegen Toxizität, die durch Chemotherapie verursacht ist, sowie die Verwendung bei Krankheiten, wobei Proteinfehlfaltung oder Aggregation ein Hauptkausalfaktor ist, wie z.B. Skrapie, Creutzfeldt-Jakob-Krankheit, Huntington oder Alzheimer (Sittler, Hum. Mol. Genet., 10, 1307, 2001; Tratzelt et al., Proc. Nat. Acad. Sci., 92, 2944, 1995; Winklhofer et al., J. Biol. Chem., 276, 45160, 2001). A. Kamal et al. beschreiben in Trends in Molecular Medicine, Vol. 10 No. 6 June 2004, therapeutische und diagnostische Anwendungen der HSP90 Aktivierung, u.a. zur Behandlung von Krankheiten des Zentralnervensystems und von Herzkreislauferkrankungen.

Die Identifikation von kleinen Verbindungen, die HSP90 spezifisch hemmen, regulieren und/oder modulieren, ist daher wünschenswert und ein Ziel der vorliegenden Erfindung.

Es wurde gefunden, daß die Verbindungen der Formel I und ihre Salze bei guter Verträglichkeit sehr wertvolle pharmakologische Eigenschaften besitzen.
Insbesondere zeigen sie inhibierende Eigenschaften des HSP90.

Gegenstand der vorliegenden Erfindung sind deshalb Verbindungen der Formel I als Arzneimittel und/oder Arzneimittelwirkstoffe bei der Behandlung und/oder Prophylaxe der genannten Erkrankungen und die Verwendung von Verbindungen der Formel I zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer Verbindungen der Formel I an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

### STAND DER TECHNIK

Andere Pyridothiophenderivate sind als HSP90-Inhibitoren in WO 2005/034950 und in WO 2005/021552 beschrieben.
In der WO 2005/00300 A1 sind Triazolderivate als HSP90-Inhibitoren beschrieben.
In der WO 00/53169 wird die HSP90-Inhibierung mit Coumarin oder einem Coumarinderivat beschrieben.
In der WO 03/041643 A2 sind HSP90-inhibierende Zearalanol-Derivate offenbart.
HSP90-inhibierende Pyrazolderivate, die in 3- oder 5-Stellung durch einen Aromaten substituiert sind, kennt man aus WO 2004/050087 A1 und WO 2004/056782 A1.
In der WO 03/055860 A1 sind 3,4-Diarylpyrazole als HSP90-Inhibitoren beschrieben.
Purinderivate mit HSP90-inhibierenden Eigenschaften sind in der WO 02/36075 A2 offenbart.

In der WO 01/72779 sind Purinverbindungen beschrieben, sowie deren Verwendung zur Behandlung von GRP94 (Homolog oder Paralog zu HSP90)-bedingten Krankheiten, wie Tumorerkrankungen, wobei das Krebsgewebe ein Sarkom oder Karzinom umfasst, ausgewählt aus der Gruppe, bestehend aus Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiosarkom, Rhabdomyosarkom, Kolonkarzinom, Pankreaskrebs, Brustkrebs, Ovarkrebs, Prostatakrebs, Plattenzellkarzinom, Basalzellkarzinom, Adenokarzinom, Schweißdrüsenkarzinom, Talgdrüsenkarzinom, Papillarkarzinom, Papillaradenokarzinomen, Cystadenokarzinomen, Knochenmarkkarzinom, bronchogenem Karzinom, Nierenzellkarzinom, Hepatom, Gallengangkarzinom, Chorionkarzinom, Seminom, embryonalem Karzinom, Wilms-Tumor, Cervix-Krebs, Hodentumor, Lungenkarzinom, kleinzelligem Lungenkarzinom, Blasenkarzinom, Epithelkarzinom, Gliom, Astrocytom, Medulloblastom, Kraniopharyngiom, Ependymom, Pinealom, Hämangioblastom, akustischem Neurom, Oligodendrogliom, Meningiom, Melanom, Neuroblastom, Retinoblastom, Leukämie, Lymphom, multiplem Myelom, Waldenströms Makroglobulinämie und Schwere-Kettenerkrankung.

In der WO 01/72779 ist weiterhin die Verwendung der dort genannten Verbindungen zur Behandlung von viralen Erkrankungen offenbart, wobei das virale Pathogen ausgewählt ist aus der Gruppe, bestehend aus Hepatitis Typ A, Hepatitis Typ B, Hepatitis Typ C, Influenza, Varicella, Adenovirus, Herpes-Simplex Typ I (HSV-I), Herpes Simplex Typ II (HSV-II), Rinderpest, Rhinovirus, Echovirus, Rotavirus, respiratorischem Synzytialvirus (RSV), Papillomvirus, Papovavirus, Cytomegalievirus, Echinovirus, Arbovirus, Huntavirus, Coxsackievirus, Mumpsvirus, Masemvirus, Rötelnvirus, Poliovirus, menschliches Immunschwächevirus Typ I (HIV-I) und menschliches Immunschwächevirus Typ II (HIV-II).
In der WO 01/72779 ist ferner die Verwendung der dort genannten Verbindungen zur GRP94-Modulation beschrieben, wobei die modulierte biologische GRP94-Aktivität eine Immunreaktion in einem Individuum, Proteintransport vom endoplasmatischen Retikulum, Genesung vom hypoxischen/anoxischen Stress, Genesung von Unterernährung, Genesung von Hitzestress, oder Kombinationen davon, hervorruft, und/oder wobei die Störung eine Art Krebs ist, eine Infektionserkrankung, eine Störung, die mit einem gestörten Proteintransport vom endoplasmatischen Retikulum, einer Störung, die mit Ischämie / Reperfusion einhergeht, oder Kombinationen davon, wobei die die mit Ischämie / Reperfusion einhergehende Störung eine Folge von Herzstillstand, Asystole und verzögerten ventrikulären Anythmien, Herzoperation, kardioputmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist.

In der WO 01/72779 ist schließlich die Verwendung einer wirksamen Menge eines GRP94-Proteinmodulators zur Herstellung eines Medikamentes bechrieben, zum Verändern einer anschließenden zellulären Reaktion auf einen ischämischen Zustand bei einer Gewebestelle in einem Individuum, durch Behandlung der Zellen an der Gewebestelle mit dem GRP94-Proteinmodulator, damit die GRP94-Aktivität in Zellen dermaßen verstärkt wird, dass eine anschließende zelluläre Reaktion auf einen ischämischen Zustand verändert wird, wobei die anschließende ischämische Bedingung vorzugsweise die Folge von Herzstillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist, oder wobei die Gewebestelle das Donatorgewebe für eine Transplantation ist.

Weitere Literatur:
Argon Y and Simen BB. 1999 "Grp94, an ER chaperone with protein and peptide binding properties", Semin. Cell Dev. Biol., Vol. 10, pp. 495-505.
Bijlmakers M-JJE, Marsh M. 2000 "Hsp90 is essential for the synthesis and subsequent membrane association, but not the maintenance, of the Srckinase p56lck", Mol. Biol. Cell, Vol. 11(5), pp. 1585-1595.
Bucci M; Roviezzo F; Cicala C; Sessa WC, Cirino G. 2000 "Geldanamycin, an inhibitor of heat shock protein 90 (Hsp90) mediated signal transduction has anti-inflammatory effects and interacts with glucocorticoid receptor in vivo", Brit. J. Pharmacol., Vol 131(1), pp. 13-16.
Carreras CW, Schirmer A, Zhong Z, Santi VS. 2003 "Filter binding assay for the geldanamycin-heat shock protein 90 interaction", Analytical Biochem., Vol 317, pp 40-46.
Chen C-F, Chen Y, Dai KD, Chen P-L, Riley DJ and Lee W-H. 1996 "A new member of the hsp90 family of molecular chaperones interacts with the retinoblastoma protein during mitosis and after heat shock", Mol. Cell. Biol., Vol. 16, pp. 4691-4699.
Chiosis G, Timaul MN, Lucas B, Munster PN, Zheng FF, Sepp-Lozenzino L and Rosen N. 2001 "A small molecule designed to bind to the adenine nucleotide pocket of HSP90 causes Her2 degradation and the growth arrest and differentiation of breast cancer cells", Chem. Biol., Vol. 8, pp. 289-299.
Chiosis G, Lucas B, Shtil A, Huezo H, Rosen N 2002 "Development of a purine-scaffold novel class of HSP90 binders that inhibit the proliferation of cancer cells and induce the degradation of her2 tyrosine kinase". Bioorganic Med. Chem., Vol 10, pp 3555-3564.
Conroy SE and Latchman DS. 1996 "Do heat shock proteins have a role in breast cancer?", Brit. J. Cancer, Vol. 74, pp. 717-721.
Felts SJ, Owen BAL, Nguyen P, Trepel J, Donner DB and Toft DO. 2000 "The HSP90-related protein TRAP1 is a mitochondrial protein with distinct functional properties", J. Biol. Chem., Vol. 5, pp. 3305-331 2.
Fuller W, Cuthbert AW. 2000 "Post-translational disruption of the delta F508 cystic fibrosis transmembrane conductance regulator (CFTR)-molecular Chaperone complex with geldanamycin stabilizes delta F508 CFTR in the rabbit reticulocyte lysate", J. Biol. Chem., Vol. 275(48), pp. 37462-37468.
Hickey E, Brandon SE, Smale G, Lloyd D and Weber LA. 1999 "Sequence and regulation of a gene encoding a human 89-kilodalton heat shock protein", Mol. Cell. Biol., Vol. 9, pp. 2615-2626.
Hoang AT, Huang J, Rudra-Gonguly N, Zheng J, Powell WC, Rabindron SK, Wu C and Roy-Burman P. 2000 "A novel association between the human heat shock transcription factor 1 (HSF1) and prostate adenocarcinoma, Am. J. Pathol., Vol. 156, pp. 857-864.
Hostein I, Robertson D, Di Stefano F, Workman P and Clarke PA. 2001 "Inhibition of signal transduction by the HSP90 inhibitor 17-allylamino-1 7-demethoxygeldanamycin results in cytostasis and apoptosis", Cancer Res., Vol. 61, pp. 4003-4009.
Hur E, Kim H-H, Choi SM, Kim JH, Yim S, Kwon HJ, Choi Y, Kim DK, Lee M-0, Park H. 2002 "Reduction of hypoxia-induced transcription through the repression of hypoxia-inducible factor-1α/aryl hydrocarbon receptor nuclear translocator DNA binding by the 90-kDa heat-shock protein inhibitor radicicol", Mol. Pharmacol., Vol 62(5), pp. 975-982.
Jameel A, Skilton RA, Campbell TA, Chander SK, Coombes RC and Luqmani YA. 1992 "Clinical and biological significance of HSP89 alpha in human breast cancer", International Journal of Cancer, Volume 50, Issue 3, pages 409-415.
Jolly C and Morimoto RI. 2000 "Role of the heat shock response and molecular chaperones in oncogenesis and cell death", J. Natl. Cancer Inst., Vol. 92, pp. 1564-1572.
Kawanishi K, Shiozaki H, Doki Y, Sakita I, Inoue M, Yano M, Tsujinata T, Shamma A and Monden M. 1999 "Prognostic significance of heat shock proteins 27 and 70 in patients with squamous cell carcinoma of the esophagus", Cancer, Vol. 85, pp. 1649-1657.
Kelland LR, Abel G, McKeage MJ, Jones M, Goddard PM, Valenti M, Murrer BA, and Harrap KR. 1993 "Preclinical antitumour evaluation of bisacetalo-amino-dichloro-cyclohexylamine platinum (IV): an orally active platinum drug", Cancer Research, Vol. 53, pp. 2581 - 2586.
Kelland LR, Sharp SY, Rogers PM, Myers TG and Workman P. 1999 "DT-diaphorase expression and tumor cell sensitivity to 17-allylamino,17-demethoxygeldanamycin, an inhibitor of heat shock protein 90", J. Natl. Cancer Inst., Vol. 91, pp. 1940-1949.
Kurebayashi J, Otsuki T, Kurosumi M, Soga S, Akinaga S, Sonoo, H. 2001 "A radicicol derivative, KF58333, inhibits expression of hypoxia-inducible factor-1α and vascular endothelial growth factor, angiogenesis and growth of human breast cancer xenografts", Jap. J. Cancer Res.,Vol. 92( 12), 1342-1351.
Kwon HJ, Yoshida M, Abe K, Horinouchi S and Bepple T. 1992 "Radicicol, an agent inducing the reversal of transformed phentoype of srctransformed fibroblasts, Biosci., Biotechnol., Biochem., Vol. 56, pp. 538-539.
Lebeau J, Le Cholony C, Prosperi MT and Goubin G. 1991 "Constitutive overexpression of 89 kDa heat shock protein gene in the HBL100 mammary cell line converted to a tumorigenic phenotype by the EJE24 Harvey-ras oncogene", Oncogene, Vol. 6, pp. 1125-1132.
Marcu MG, Chadli A, Bouhouche I, Catelli M and Neckers L. 2000a "The heat shock protein 90 antagonist novobiocin interacts with a previously unrecognized ATP-binding domain in the carboxyl terminus of the chaperone", J. Biol. Chem., Vol. 275, pp. 37181-37186.
Marcu MG, Schulte TW and Neckers L. 2000b "Novobiocin and related coumarins and depletion of heat shock protein 90-dependent signaling proteins", J. Natl. Cancer Inst., Vol. 92, pp. 242-248.
Martin KJ, Kritzman BM, Price LM, Koh B, Kwan CP, Zhang X, MacKay A, O'Hare MJ, Kaelin CM, Mutter GL, Pardee AB and Sager R. 2000 "Linking gene expression pattems to therapeutic groups in breast cancer", Cancer Res., Vol. 60, pp. 2232-2238.
Neckers L, Schulte TW and Momnaaugh E. 1999 "Geldanamycin as a potential anti-cancer agent: its molecular target and biochemical activity", Invest. New Druqs, Vol. 17, pp. 361-373.
Page J, Heath J, Fulton R, Yalkowsky E, Tabibi E, Tomaszewski J, Smith A and Rodman L. 1997 "Comparison of geldanamycin (NSC-122750) and 17-allylaminogeldanamycin (NSC-330507D) toxicity in rats", Proc. Am. Assoc. Cancer Res., Vol. 38, pp. 308.
Panaretou B, Prodromou C, Roe SM, OBrien R, Ladbury JE, Piper PW and Pearl LH. 1998 "ATP binding and hydrolysis are essential to the function of the HSP90 molecular chaperone in vivo", EMBO J., Vol. 17, pp. 4829-4836.
Pratt WB. 1997 "The role of the HSP90-based chaperone system in signal transduction by nuclear receptors and receptors signalling via MAP kinase", Annu. Rev. Pharmacol. Toxicol., Vol. 37, pp. 297-326.
Prodromou C, Roe SM, O'Brien R, Ladbury JE, Piper PW and Pearl LH. 1997 "Identification and structural characterization of the ATP/ADP-binding site in the HSP90 molecular chaperone", Cell, Vol. 90, pp. 65-75.
Prodromou C, Panaretou B, Chohan S, Siligardi G, O'Brien R, Ladbury JE, Roe SM, Piper PW and Pearl LH. 2000 "The ATPase cycle of HSP90 drives a molecular "clamp" via transient dimerization of the N-terminal domains", EMBO J., Vol. 19, pp. 4383-4392.
Roe SM, Prodromou C, O'Brien R, Ladbury JE, Piper PW and Pearl LH. 1999 "Structural basis for inhibition of the HSP90 molecular chaperone by the antitumour antibiotics radicicol and geldanamycin", J. Med. Chem., Vol. 42, pp. 260-266.
Rutherford SL and Lindquist S. 1998 "HSP90 as a capacitor for morphological evolution. Nature, Vol. 396, pp. 336-342.
Schulte TW, Akinaga S, Murakata T, Agatsuma T, Sugimoto S, Nakano H, Lee YS, Simen BB, Argon Y, Felts S, Toft DO, Neckers LM and Sharma SV. 1999 "Interaction of radicicol with members of the heat shock protein 90 family of molecular chaperones", Mol. Endocrinoloqy, Vol. 13, pp. 1435-1448.
Schulte TW, Akinaga S, Soga S, Sullivan W, Sensgard B, Toft D and Neckers LM. 1998 "Antibiotic radicicol binds to the N-terminal domain of HSP90 and shares important biologic activities with geldanamcyin", Cell Stress and Chaperones, Vol. 3, pp. 100-108.
Schulte TW and Neckers LM. 1998 "The benzoquinone ansamycin 17-allylamino-17-demethoxygeldanamcyin binds to HSP90 and shares important biologic activities with geldanamycin", Cancer Chemother. Pharmacol., Vol. 42, pp. 273-279.
Smith DF. 2001 "Chaperones in signal transduction", in: Molecular chaperones in the cell (P Lund, ed.; Oxford University Press, Oxford and NY), pp. 165-178.
Smith DF, Whitesell L and Katsanis E. 1998 "Molecular chaperones: Biology and prospects for pharmacological intervention", Pharmacological Reviews, Vol. 50, pp. 493-513.
Song HY, Dunbar JD, Zhang YX, Guo D and Donner DB. 1995 "Identification of a protein with homology to hsp90 that binds the type 1 tumour necrosis factor receptor", J. Biol. Chem., Vol. 270, pp. 3574-3581.
Stebbins CE, Russo A, Schneider C, Rosen N, Hartl FU and Pavletich NP. 1997 "Crystal structure of an HSP90-geldanamcyin complex: targeting of a protein chaperone by an antitumor agent", Cell, Vol. 89, pp. 239-250.
Supko JG, Hickman RL, Grever MR and Malspeis L. 1995 "Preclinical pharmacologic evaluation of geldanamycin as an antitumour agent", Cancer Chemother. Pharmacol., Vol. 36, pp. 305-315.
Tytell M and Hooper PL. 2001 "Heat shock proteins: new keys to the development of cytoprotective therapies", Emerging Therapeutic Tarqets, Vol. 5, pp. 267-287.
Uehara U, Hori M, Takeuchi T and Umezawa H. 1986 "Phenotypic change from transformed to normal induced by benzoquinoid ansamycins accompanies inactivation of p6Osrc in rat kidney cells infected with Rous sarcoma virus", Mol. Cell. Biol., Vol. 6, pp. 21 98-2206.
Waxman, Lloyd H. Inhibiting hepatitis C virus processing and replication. (Merck & Co., Inc., USA). PCT Int. Appl. (2002), WO 0207761 Whitesell L, Mimnaugh EG, De Costa B, Myers CE and Neckers LM. 1994 "Inhibition of heat shock protein HSP90-pp60v-src heteroprotein complex formation by benzoquinone ansamycins: essential role for stress proteins in oncogenic transformation", Proc. Natl. Acad. Sci. USA., Vol. 91, pp. 8324-8328.
Yorgin et al. 2000 "Effects of geldanamycin, a heat-shock protein 90-binding agent, on T cell function and T cell nonreceptor protein tyrosine kinases", J. Immunol., Vol 164(6), pp. 2915-2923.
Young JC, Moarefi I and Hartl FU. 2001 "HSP90: a specialized but essential protein-folding tool", J. Cell. Biol., Vol. 154, pp. 267-273.
Zhao JF, Nakano H and Sharma S. 1995 "Suppression of RAS and MOS transformation by radicicol", Oncoqene, Vol. 11, pp. 161 -173.

### ZUSAMMENFASSUNG DER ERFINDUNG

Die Erfindung betrifft Verbindungen der Formel I 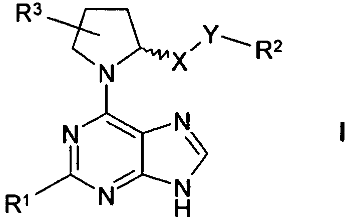 worin
- X: CH₂ oder CO,
- Y: O, S, NH(CH₂)ₙ oder CH₂,
- R¹: H, A oder Hal,
- R²: Ar oder Het,
- R³: H, OH, (CH₂)ₙOH, oder (CH₂)ₚCH(OH)(CH₂)ₚOH,
- A, A': jeweils unabhängig voneinander unverzweigtes oder verzweigtes Alkyl mit 1-10 C-Atomen, worin 1-5 H-Atome durch F, Cl und/oder Br ersetzt sein können,
Alk oder cyclisches Alkyl mit 3-7 C-Atomen,
- A und A': zusammen auch eine Alkylenkette mit 2, 3, 4, 5 oder 6 C-Atomen, worin eine oder zwei CH₂-Gruppen durch O, S, SO, SO₂, NH, NA und/oder N-COOA ersetzt sein können,
- Alk: Alkenyl mit 2-6 C-Atomen,
- Ar: unsubstituiertes oder ein-, zwei-, drei-, vier- oder fünffach durch A, OA, OH, SH, SA, Hal, NO₂, CN, (CH₂)ₙAr', (CH₂)ₙCOOH, (CH₂)ₙCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA',NACONAA', NHCO(CH₂)ₙNH₂ und/oder -O-(CH₂)ₒ-Het¹ substituiertes Phenyl, Naphthyl oder Biphenyl,
- Ar': unsubstituiertes oder ein-, zwei- oder dreifach durch A, OA, OH, SH, SA, Hal, NO₂, CN, (CH₂)ₙPhenyl, (CH₂)ₙCOOH, (CH₂)ₙCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA' und/oder NACONAA' substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der ein-, zwei- oder dreifach durch A, OA, OH, SH, SA, Hal, NO₂, CN, (CH₂)ₙAr', (CH₂)ₙCOOH, (CH₂)ₙCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA', NACONAA', SO₂A, =S, =NH, =NA und/oder =O (Carbonylsauerstoff) substituiert sein kann,
- Het¹: einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
- Hal: F, Cl, Br oder I,
- m: 2, 3, 4, 5 oder 6
- n: 0, 1, 2, 3 oder 4,
- o: 1 oder 2,
- p: 1, 2, 3 oder 4,
bedeuten,
sowie ihre pharmazeutisch verwendbaren Salze, Solvate, Tautomere und Stereoisomere, einschließlich deren Mischungen in allen Verhältnissen.

Gegenstand der Erfindung sind die Verbindungen der Formel I und ihre Salze sowie ein Verfahren zur Herstellung von Verbindungen der Formel I nach den Ansprüchen 1-14 sowie ihrer pharmazeutisch verwendbaren Solvate, Salze, Tautomere und Stereoisomere, dadurch gekennzeichnet, daß man eine Verbindung der Formel II 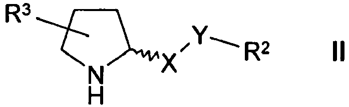 worin
X, Y, R² und R³ die in Anspruch 1 angegebenen Bedeutungen haben,
mit
einer Verbindung der Formel III 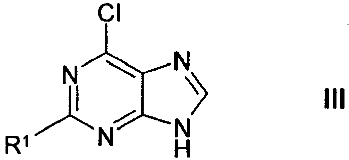 worin R¹ die in Anspruch 1 angegebene Bedeutung hat,
umsetzt,
und/oder eine Base oder Säure der Formel I in eines ihrer Salze umwandelt.

Gegenstand der Erfindung sind auch die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Die erfindungsgemäßen Verbindungen der Formel I können auch in tautomeren Formen vorliegen. Formel I umfaßt alle diese tautomeren Formen.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, gilt, daß deren Bedeutungen unabhängig voneinander sind.
Vor- und nachstehend haben die Reste bzw. Parameter R¹, R², R³, X und Y die bei der Formel I angegebenen Bedeutungen, falls nicht ausdrücklich etwas anderes angegeben ist.

A bzw. A' bedeutet vorzugsweise Alkyl, ist unverzweigt (linear) oder verzweigt, und hat 1, 2, 3, 4, 5, 6, 7, 8, 9 oder 10 C-Atome. A bzw. A' bedeutet besonders bevorzugt Methyl, Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.
A bzw. A' bedeutet ganz besonders bevorzugt Alkyl mit 1, 2, 3, 4, 5 oder 6 C-Atomen, vorzugsweise Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl, ferner auch Fluormethyl, Difluormethyl oder Brommethyl.
A bzw. A' bedeutet auch Cycloalkyl. Cycloalkyl bedeutet vorzugsweise Cyclopropyl, Cyclobutyl, Cylopentyl, Cyclohexyl oder Cycloheptyl.
A bzw. A' bedeutet auch Alk. Alk bedeutet Alkenyl mit 2-6 C-Atomen, wie z.B. Vinyl oder Propenyl.
Cycloalkylalkylen bedeutet z. B. Cyclohexylmethyl, Cyclohexylethyl, Cyclopentylmethyl oder Cyclopentylethyl.

Ac bedeutet Acetyl, Bzl bedeutet Benzyl, Ms bedeutet -SO₂CH₃.

X bedeutet vorzugsweise CH₂, ferner CO.
Y bedeutet vorzugsweise O oder NH(CH₂)ₙ, ferner S oder CH₂.

R¹ bedeutet vorzugsweise H.
R³ bedeutet vorzugsweise H, OH, oder (CH₂)ₚCH(OH)(CH₂)ₚOH.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m-oder p-Aminophenyl, o-, m- oder p-(N-Methylamino)-phenyl, o-, m- oder p-(N-Methylaminocarbonyl)-phenyl, o-, m- oder p-Acetamidophenyl, o-, m-oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethy)armno)-phenyt, o-, m- oder p-Fluorphenyl, o-, m-oder p-Bromphenyl, o-, m- oder p- Chlorphenyl, o-, m- oder p-(Methyl-sulfonamido)-phenyl, o-, m- oder p-(Methylsulfonyl)-phenyl, o-, m- oder p-Cyanphenyl, o-, m- oder p-Ureidophenyl, o-, m- oder p-Formylphenyl, o-, m- oder p-Acetylphenyl, o-, m- oder p-Aminosulfonylphenyl, o-, m- oder p-Carboxyphenyl, o-, m- oder p-Carboxymethyl-phenyl, o-, m- oder p-Carboxymethoxy-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlor-phenyl, 2-Nitro-4-N,N-dimethylamino- oder 3-Nitro-4-N,N-dimethylamino-phenyl, 2,3-Diaminophenyl, 2,3,4-, 2,3,5-, 2,3,6-, 2,4,6- oder 3,4,5-Tri-chlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet vorzugsweise unsubstituiertes oder ein-, zwei-, drei-, vier- oder fünffach durch A, Hal, OA, OH oder CN substituiertes Phenyl.

Ar' bedeutet vorzugsweise z.B. unsubstituiertes oder ein-, zwei- oder dreifach durch Hal substituiertes Phenyl.

Het bedeutet, ungeachtetet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4-oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4-oder 5-Benzimidazolyl, 1-, 2-, 3-, 4-, 5-, 6- oder 7-Indazolyl, 1-, 3-, 4-, 5-, 6-oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7-oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxo-lan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3-oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet vorzugsweise einen einkernigen aromatischen Heterocyclus mit 1 bis 3 N-Atomen, wie z.B. Pyridyl, Pyrimidinyl, Pyrazolyl, Triazolyl, Tetrazoly, Imidazolyl, Pyridazinyl oder Pyrazinyl, der ein-, zwei- oder dreifach durch A, OA und/oder Hal substituiert sein kann.

Het¹ bedeutet vorzugsweise einen einkernigen gesättigten Heterocyclus mit 1 bis 2 N- und/oder O-Atomen, der ein- oder zweifach durch A und/oder =O (Carbonylsauerstoff) substituiert sein kann, besonders bevorzugt ist 4-Methyl-piperazinyl.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis If ausgedrückt werden, die der Formel I entsprechen und worin die nicht näher bezeichneten Reste die bei der Formel I angegebene Bedeutung haben, worin jedoch
in Ia
- Y: O oder NH(CH₂)ₙ bedeutet;
in Ib
- R³: H,OH,oder (CH₂)ₚCH(OH)(CH₂)ₚOH bedeutet;
in Ic
- Ar: unsubstituiertes oder ein-, zwei-, drei-, vier- oder fünffach durch A, Hal, OA, OH oder CN substituiertes Phenyl,
bedeuten;
in Id
- Het: einen einkernigen aromatischen Heterocyclus mit 1 bis 3 N-Atomen, der ein-, zwei- oder dreifach durch A, OA und/oder Hal substituiert sein kann, bedeutet;
in Ie
- A: unverzweigtes oder verzweigtes Alkyl mit 1-6 C-Atomen, worin 1-5 H-Atome durch F und/oder Cl ersetzt sein können,
oder Alk,
bedeutet;
in If
- X: CH₂ oder CO,
- Y: O oder NH(CH₂)ₙ,
- R¹: H,
- R²: Ar oder Het,
- R³: H, OH, oder (CH₂)ₚCH(OH)(CH₂)ₚOH,
- A: unverzweigtes oder verzweigtes Alkyl mit 1-6 C-Atomen, worin 1-5 H-Atome durch F und/oder Cl ersetzt sein können,
oder Alk,
- Alk: Alkenyl mit 2-6 C-Atomen,
- Ar: unsubstituiertes oder ein-, zwei-, drei-, vier- oder fünffach durch A, Hal, OA, OH oder CN substituiertes Phenyl,
- Het: einen einkernigen aromatischen Heterocyclus mit 1 bis 3 N-Atomen, der ein-, zwei- oder dreifach durch A, OA und/oder Hal substituiert sein kann,
- Hal: F, Cl, Br oder I,
- p: 1, 2, 3 oder 4,
- n: 0, 1 oder 2
bedeuten;
sowie ihre pharmazeutisch verwendbaren Solvate, Salze, Tautomere und Stereoisomere, einschließlich deren Mischungen in allen Verhältnissen.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Verbindungen der Formel I können vorzugsweise erhalten werden, indem man eine Verbindung der Formel II mit einer Verbindung der Formel III umsetzt.

Die Verbindungen der Formel II und III sind in der Regel bekannt. Sind sie nicht bekannt, so können sie nach an sich bekannten Methoden hergestellt werden.

Die Umsetzung erfolgt nach Methoden, die dem Fachmann bekannt sind.

Die Reaktion erfolgt in einem geeigneten inerten Lösungsmittel.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chlorform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Als Lösungsmittel besonders bevorzugt ist z.B. Aceton, Wasser und/oder Tetrahydrofuran.

Die Umsetzung kann gegebenenfalls unter basischen Bedingungen erfolgen. Als Basen eignen sich vorzugsweise Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin oder Diethanolamin.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 130°, insbesondere zwischen etwa 30° und etwa 125°.

Es ist ferner möglich, eine Verbindung der Formel I in eine andere Verbindung der Formel I umzuwandeln, indem man einen oder mehrere Rest(e) R² und/oder R³ in einen oder mehrere Rest(e) R² und/oder R³ umwandelt, z.B. indem man Nitrogruppen, beispielsweise durch Hydrierung an Raney-Nickel oder Pd-Kohle in einem inerten Lösungsmittel wie Methanol oder Ethanol, zu Aminogruppen reduziert und/oder eine Estergruppe in eine Carboxygruppe umwandelt und/oder eine Aminogruppe durch reduktive Aminierung in ein alkyliertes Amin umwandelt und/oder
Carboxygruppen durch Umsetzung mit Alkoholen verestert und/oder Säurechloride durch Umsetzung mit einem Amin in ein Säureamid überführt und/oder
eine Hydroxygruppe z.B. mit einem Alkylhalogenid alkyliert.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind. COOH-Gruppen werden bevorzugt in Form ihrer tert.-Butylester geschützt.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5n HCI in Dioxan bei 15-30° abgespalten werden, die FMOC-Gruppe mit einer etwa 5- bis 50 %igen Lösung von Dimethylamin, Diethylamin oder Piperidin in DMF bei 15-30°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydröxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinät, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, lsethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der Verbindungen der Formel I unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Salze Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,1 mg bis 3 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein. Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendem mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältem, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der Verbindung der Formel I *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Salze, Solvate und Stereoisomere, einschließlich deren Mischungen in allen Verhältnissen, und mindestens einen weiteren Arzneimittelwirkstoff.

Als weitere Arzneimittelwirkstoffe sind Chemotherapeutika bevorzugt, insbesondere solche, die Angiogenese hemmen und dadurch das Wachstum und die Verbreitung von Tumorzellen inhibieren; bevorzugt sind dabei VEGF-Rezeptorinhibitoren, beinhaltend Robozyme und Antisense, die auf VEGF-Rezeptoren gerichtet sind, sowie Angiostatin und Endostatin.

Beispiele antineoplastischer Agenzien, die in Kombination mit den erfindungsgemäßen Verbindungen verwendet werden können, beinhalten im allgemeinen alkylierende Agenzien, Antimetaboliten; Epidophyllotoxin; ein antineoplastisches Enzym; einen Topoisomerase-Inhibitor; Procarbazin; Mitoxantron oder Platin-Koordinationskomplexe.

Antineoplastische Agenzien sind vorzugsweise ausgewählt aus den folgenden Klassen:
Anthracycline, Vinca-Arzneistoffe, Mitomycine, Bleomycine, cytotoxische Nukleoside, Epothilone, Discodermolide, Pteridine, Diynene und Podophyllotoxine.
Besonders bevorzugt sind in den genanten Klassen z.B. Carminomycin, Daunorubicin, Aminopterin, Methotrexat, Methopterin, Dichlormethotrexat, Mitomycin C, Porfiromycin, 5-Fluoruracil, 5-Fluordeoxyuridin Monophosphat, Cytarabine, 5-Azacytidin, Thioguanin, Azathioprine, Adenosin, Pentostatin, Erythrohydroxynonyladenin, Cladribine, 6-Mercaptopurin, Gemcitabine, Cytosinarabinosid, Podophyllotoxin oder Podophyllotoxinderivate, wie z.B. Etoposide, Etoposide Phosphat oder Teniposide, Melphalan, Vinblastine, Vinorelbine, Vincristine, Leurosidine, Vindesine, Leurosine, Docetaxel und Paclitaxel. Andere bevorzugte antineoplastische Agenzien sind ausgewählt aus der Gruppe Discodermolide, Epothilone D, Estramustine, Carboplatin, Cisplatin, Oxaliplatin, Cyclophosphamid, Bleomycin, Gemcitabine, Ifosamide, Melphalan, Hexamethylmelamin, Thiotepa, Idatrexate, Trimetrexate, Dacarbazine, L-Asparaginase, Camptothecin, CPT-11, Topotecan, Arabinosyl-Cytosin, Bicalutamide, Flutamide, Leuprolide, Pyridobenzoindolderivate, Interferone und Interleukine.

Als weitere Arzneimittelwirkstoffe sind Antibiotica bevorzugt. Bevorzugte Antibiotica sind ausgewählt aus der Gruppe Dactinomycin, Daunorubicin, Idarubicin, Epirubicin, Mitoxantrone, Bleomycin, Plicamycin, Mitomycin.

Als weitere Arzneimittelwirkstoffe sind Enzyminhibitoren bevorzugt. Bevorzugte Enzyminhibitoren sind ausgewählt aus der Gruppe der Histon-Deacetylierungs-Inhibitoren (z.B. suberoylanilide hydroxamic acid [SAHA]) und der Tyrosinkinase-Inhibitoren (z.B. ZD 1839 [Iressa]).

Als weitere Arzneimittelwirkstoffe sind Nuclear-Export-Inhibitoren bevorzugt. Nuclear-Export-Inhibitoren verhindern die Ausschleusung von Biopolymeren (z.B. RNA) aus dem Zellkern. Bevorzugte Nuclear-Export-Inhibitoren sind ausgewählt aus der Gruppe Callystatin, Leptomycin B, Ratjadone.

Als weitere Arzneimittelwirkstoffe sind Nuclear-Export-Inhibitoren bevorzugt. Nuclear-Export-Inhibitoren verhindern die Ausschleusung von Biopolymeren (z.B. RNA) aus dem Zellkern. Bevorzugte Nuclear-Export-Inhibitoren sind ausgewählt aus der Gruppe Callystatin, Leptomycin B, Ratjadone.

Als weitere Arzneimittelwirkstoffe sind Immunsuppressiva bevorzugt. Bevorzugte Immunsuppressiva sind ausgewählt aus der Gruppe Rapamycin, CCI-779 (Wyeth), RAD001 (Novartis), AP23573 (Ariad Pharmaceuticals).

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Salze, Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von Krankheiten, bei denen HSP90 eine Rolle spielt.

Gegenstand der Erfindung ist somit die Verwendung von Verbindungen der Formel I, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation von HSP90 eine Rolle spielt.

Die vorliegende Erfindung umfasst die Verwendung der Verbindungen der Formel I und/oder ihre physiologisch unbedenklichen Salze und Solvate zur Herstellung eines Arzneimittels zur Behandlung von Tumorerkrankungen, wie z.B. Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiosarkom, Rhabdomyosarkom, Kolonkarzinom, Pankreaskrebs, Brustkrebs, Ovarkrebs, Prostatakrebs, Plattenzellkarzinom, Basalzellkarzinom, Adenokarzinom, Schweißdrüsenkarzinom, Talgdrüsenkarzinom, Papillarkarzinom, Papillaradenokarzinomen, Cystadenokarzinomen, Knochenmarkkarzinom, bronchogenem Karzinom, Nierenzellkarzinom, Hepatom, Gallengangkarzinom, Chorionkarzinom, Seminom, embryonalem Karzinom, Wilms-Tumor, Cervix-Krebs, Hodentumor, Lungenkarzinom, kleinzelligem Lungenkarzinom, Blasenkarzinom, Epithelkarzinom, Gliom, Astrocytom, Medulloblastom, Kraniopharyngiom, Ependymom, Pinealom, Hämangioblastom, akustischem Neurom, Oligodendrogliom, Meningiom, Melanom, Neuroblastom, Retinoblastom, Leukämie, Lymphom, multiplem Myelom, Waldenströms Makroglobulinämie und Schwere-Kettenerkrankung; viralen Erkrankungen, wobei das virale Pathogen ausgewählt ist aus der Gruppe, bestehend aus Hepatitis Typ A, Hepatitis Typ B, Hepatitis Typ C, Influenza, Varicella, Adenovirus, Herpes-Simplex Typ I (HSV-I), Herpes Simplex Typ II (HSV-II), Rinderpest, Rhinovirus, Echovirus, Rotavirus, respiratorischem Synzytialvirus (RSV), Papillomvirus, Papovavirus, Cytomegalievirus, Echinovirus, Arbovirus, Huntavirus, Coxsackievirus, Mumpsvirus, Masemvirus, Rötelnvirus, Poliovirus, menschliches lmmunschwächevirus Typ I (HIV-I) und menschliches immunschwächevirus Typ II (HIV-II);
zur Immunsuppression bei Transplantationen; entzündungsbedingten Erkrankungen, wie Rheumatoide Arthritis, Asthma, Multiple Sklerose, Typ 1 Diabetes, Lupus Erythematodes, Psoriasis und Inflammatory Bowel Disease; Zystische Fibrose; Erkrankungen im Zusammenhang mit Angiogenese wie z.B. diabetische Retinopathie, Hämangiome, Endometriose, Tumorangiogenese; infektiösen Erkrankungen; Autoimmunerkrankungen; Ischämie; Förderung der Nervenregeneration; fibrogenetischen Erkrankungen, wie z.B. Sklerodermie, Polymyositis, systemischer Lupus, Leberzirrhose, Keloidbildung, interstitielle Nephritis und pulmonare Fibrose;

Die Verbindungen der Formel I können insbesondere das Wachstum von Krebs, Tumorzellen und Tumormetastasen hemmen und sind deshalb für die Tumortherapie geeignet.

Die vorliegende Erfindung umfasst weiterhin die Verwendung der Verbindungen der Formel I und/oder ihre physiologisch unbedenklichen Salze und Solvate zur Herstellung eines Arzneimittels zum Schutz normaler Zellen gegen Toxizität, die durch Chemotherapie verursacht ist, sowie zur Behandlung von Krankheiten, wobei Proteinfehlfaltung oder Aggregation ein Hauptkausalfaktor ist, wie z.B. Skrapie, Creutzfeldt-Jakob-Krankheit, Huntington oder Alzheimer.

Die Erfindung betrifft auch die Verwendung der Verbindungen der Formel I und/oder ihre physiologisch unbedenklichen Salze und Solvate zur Herstellung eines Arzneimittels zur Behandlung von Krankheiten des Zentralnervensystems, von Herzkreislauferkrankungen und Kachexie.

Die Erfindung betrifft in einer weiteren Ausführungsform auch die Verwendung der Verbindungen der Formel I und/oder ihre physiologisch unbedenklichen Salze und Solvate zur Herstellung eines Arzneimittels zur HSP90-Modulation, wobei die modulierte biologische HSP90-Aktivität eine Immunreaktion in einem Individuum, Proteintransport vom endoplasmatischen Retikulum, Genesung vom hypoxischen/anoxischen Stress, Genesung von Unterernährung, Genesung von Hitzestress, oder Kombinationen davon, hervorruft, und/oder wobei die Störung eine Art Krebs ist, eine Infektionserkrankung, eine Störung, die mit einem gestörten Proteintransport vom endoplasmatischen Retikulum, einer Störung, die mit Ischämie / Reperfusion einhergeht, oder Kombinationen davon, wobei die die mit Ischämie / Reperfusion einhergehende Störung eine Folge von Herzstillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist.

Die Erfindung betrifft in einer weiteren Ausführungsform auch die Verwendung der Verbindungen der Formel I und/oder ihre physiologisch unbedenklichen Salze und Solvate zur Herstellung eines Arzneimittels zur Behandeln von Ischämie als Folge von Herzstillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist.

### Testverfahren zur Messung von HSP90 Inhibitoren

Die Bindung von Geldanamycin oder 17- Allylamino-17-demethoxygeldanamycin (17AAG) und deren kompetitive Hemmung an HSP90 kann benutzt werden, um die inhibitorische Aktivität der erfindungsgemäßen Verbindungen zu bestimmen (Carreras et al. 2003, Chiosis et al. 2002). Im speziellen Fall wird ein Radioligand-Filterbindungstest verwendet. Als Radioligand wird dabei mit Tritium markiertes 17-Allylamino-geldanamycin, [3H]17AAG, verwendet. Dieser Filter-Bindungstest erlaubt eine gezielte Suche nach Inhibitoren, die mit der ATP-Bindestelle interferieren.

### Material

Rekombinantes humanes HSP90α (E. coli exprimiert, 95% Reinheit); [3H]17AAG (17-Allylamino-geldanamycin, [allylamino-2,3-³H. Spezifische Aktivität: 1,11x10¹² Bq/mmol (Moravek, MT-1717);
HEPES Filterpuffer (50 mM HEPES, pH 7,0, 5mM MgCl2, BSA 0.01 %) Multiscreen-FB (1µm) Filterplatte (Millipore, MAFBNOB 50).

### Methode

Die 96 well Mikrotiter-Filterplatten werden zunächst gewässert und mit 0,1% Polyethylenimin beschichtet.

Der Test wird unter folgenden Bedingungen durchgeführt:
Reaktionstemperatur 22 °C
Reaktionszeit: 30 min., Schütteln bei 800 upm
Testvolumen: 50 µl
Endkonzentrationen:
50 mM HEPES-HCl, pH7,0, 5 mM MgCl2, 0,01 % (w/v) BSA
HSP90: 1,5 µg/assay
[3H]17AAG: 0,08 µM.

Am Ende der Reaktion wird der Überstand in der Filterplatte mit Hilfe eines Vakuum-Manifolds (Multiscreen Separation System, Millipore) abgesaugt und der Filter zweimal gewaschen.
Die Filterplatten werden dann in einem Beta-counter (Microbeta, Wallac) mit Szintillator (Microscint 20, Packard) gemessen.

Aus den "counts per minutes"-Werten wird "% der Kontrolle" ermittelt und daraus der IC-50 Wert einer Verbindung kalkuliert.

**Tabelle I**

| HSP90-Inhibierung durch erfindungsgemäße Verbindungen | |
|---|---|
| Verbindung der Formel I | IC₅₀ [mol/l] |
| "A47" | 9.0 x 10⁻⁷ |
| "A52" | 2.0 x 10⁻⁵ |
| "A53" | 3.0 x 10⁻⁵ |
| "A57" | 1.5 x 10⁻⁶ |
| "A58" | 6.3 x 10⁻⁶ |

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

### LC-MS Bedingungen

Hewlett Packard System der HP 1100 Serie mit den folgenden Merkmalen: lonenquelle: Elektrospray (positive mode); Scan: 100-1000 m/z;
Fragmentier-Spannung: 60 V; Gas-Temperatur: 300°C, DAD: 220 nm.
Flussrate: 2.4 ml/Min. Der verwendete Splitter reduzierte nach dem DAD die Flussrate für das MS auf 0,75ml/Min.
Säule: Chromolith SpeedROD RP-18e 50-4.6
Lösungsmittel: LiChrosolv-Qualität der Fa. Merck KGaA

### Gradienten:

| | |
|---|---|
| P1: | Lösungsmittel A: H2O (0.1% TFA) |
| | Lösungsmittel B: ACN (0.1% TFA) |
| | 99% A → 100% B: Laufzeit 6 Minuten / Flußrate 3,0 ml/Minute |
| P2: | Lösungsmittel A: H2O (0.01 % TFA) |
| | Lösungsmittel B: ACN (0.01 % TFA) |
| | 99% A → 100% B: Laufzeit 5 Minuten / Flußrate 2,75 ml/Minute |
| N : | Lösungsmittel A: H2O (0.01% TFA) |
| | Lösungsmittel B: ACN (0.01% TFA) |
| | 90% A → 100% B: Laufzeit 5 Minuten / Flußrate 2,75 ml/Minute |

Die in den nachfolgenden Beispielen angegebenen Retentionszeiten RT [min] und M+H⁺-Daten MW sind die Meßergebnisse der LC-MS-Messungen.

### Beispiel 1

### Herstellung von 6-[(R)-2-(5-Fluor-2-methoxy-phenoxymethyl)-pyrrolidin-1-yl]-9H-purin ("A1")

1.1 Die Umsetzung erfolgt unter Stickstoffatmosphäre. Zu einer Lösung von 83,77 g (R)-Prolinmethylester in 600 ml tert.-Butanol gibt man 151 ml Triethylamin und anschließend 300 ml tert.-Butanol. Danach tropft man eine Lösung von 96,03 g Di-tert.-butyldicarbonat [(BOC)₂O] in 100 ml tert.-Butanol zu und rührt 20 Stunden bei Raumtemperatur. Der ausgefallene Niederschlag wird abgetrennt. Das Produkt befindet sich im Filtrat. Man entfernt das Lösungsmittel und nimmt den Rückstand in 700 ml Diethylether auf. Man wäscht mit 2 x 500 ml 1 N HCl, 1 x mit 500 ml gesättigter Na₂CO₃-Lösung und 1 x mit 500 ml gesättigter NaCl-Lösung. Nach Trocknen über Na₂SO₄ wird das Lösungsmittel entfernt. Man erhält 86,7 g BOC-(R)-Prolinmethylester ("1") als gelbes Öl.
1.2 1,22 g Lithiumchlorid wird mit 14 ml Ethanol versetzt und 10 Minuten gerührt. Man kühlt auf -20° ab, tropft eine Suspension von 1,09 g Natriumborhydrid in 14 ml Ethanol zu und rührt noch 15 Minuten bei -20°. Anschließend wird eine Lösung von 3,0 g "1" in 14 ml THF zugetropft. Man rührt noch.30 Minuten bei -20° und 19 Stunden bei Raumtemperatur. Das Gemisch wird auf 0° gekühlt und mit 10 %iger Citronensäurelösung versetzt bis ein pH von 3-4 erreicht ist. Man extrahiert dreimal mit 100 ml Ethylacetat. Die organischen Phasen werden wie üblich aufgearbeitet. Man erhält 2,57 g BOC-(R)-Prolinol ("2"); MS [M+1 -BOC]: 102.
1.3 694 mg "2", 539 mg 5-Fluor-2-methoxy-phenol, 2,29 g Triphenylphosphin (polymer gebunden) und 0,48 ml Triethylamin werden in 3 ml THF in einem Mikrowellengefäß suspendiert. Man spült mit Stickstoff und verschließt mit einem Septum. Dann gibt man eine Lösung von 1,19 g Di-tert.-butyl-azodicarboxylat in 2 ml THF zu und erhitzt 10 Minuten bei 125° in der Mikrowelle. Der ausgefallene Niederschlag wird abgetrennt. Zum Filtrat gibt man 6 g Ionenaustauscher (III, stark basisch, OH-Form, LA 104767) und rührt 30 Minuten bei Raumtemperatur. Dann wird der Ionenaustauscher abgetrennt. Nach Abtrennen des Lösungsmittels vom Filtrat erhält man (R)-2-(5-fluor-2-methoxy-phenoxymethyl)-*N*-BOC-pyrrolidin ("5") 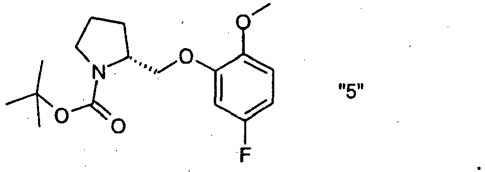
1.4 Eine Lösung von 2,32 g "5" in 150 ml Dichlormethan wird mit 30 ml Trifluoressigsäure versetzt und 16 Stunden bei Raumtemperatur stehen gelassen. Man versetzt mit 150 ml Wasser und extrahiert die organische Phase 3 x mit je 200 ml 1 N HCl. Die organische Phase wird verworfen. Die wässrige Phase wird mit 0,1 M K₃PO₄ auf pH 12 gestellt und 4 x mit je 200 ml Ethylacetat extrahiert. Die organische Phase wird über Na₂SO₄ getrocknet. Nach Entfernen des Lösungsmittels erhält man 1,49 g (R)-2-(5-fluor-2-methoxy-phenoxymethyl)-pyrrolidin ("6").
1.5 Eine Lösung von 500 mg "6" und 165,5 mg 6-Chlorpurin in 2,5 ml Aceton wird bei 150° in der Mikrowelle erhitzt. Das Lösungsmittel wird abgetrennt und der Rückstand wird über HPLC chromatographiert. Man erhält 125 mg 6-[(R)-2-(5-Fluor-2-methoxy-phenoxymethyl)-pyrrolidin-1-yl]-9*H*-purin ("A1"); HPLC Retentionszeit 2,83 Minuten; Gradient P1; [M+H]⁺ 344,

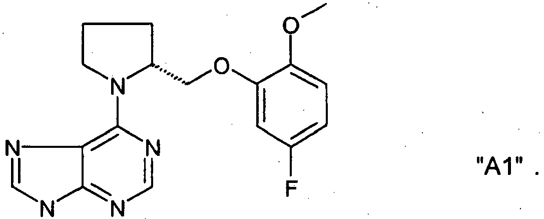

### Beispiel A

### Herstellung weiterer Zwischenverbindungen

A-1 Zu einer Lösung von 300 mg "2" in 5 ml Dichlormethan gibt man 247,9 µl Triethylamin, tropft dann 127,1 µl Methansulfonylchlorid zu und rührt 45 Minuten bei Raumtemperatur nach. Man arbeitet wie üblich auf und erhält 392 mg (R)-2-Methansulfonyloxymethyl-*N*-BOC-pyrrolidin ("3")

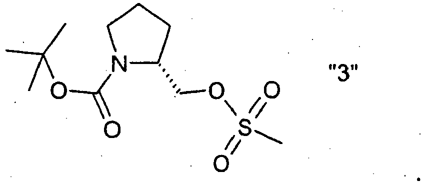

A-2 Eine Lösung von 247 mg "2" und 343,9 mg Pyridiniumchlorochromat in 4 ml Dichlormethan wird 18 Stunden bei Raumtemperatur gerührt. Nach Abtrennen des ausgefallenen Niederschlags wird das Filtrat noch zweimal mit je 3 ml Dichlormethan versetzt und der ausgefallene Niederschlag wird jeweils abgetrennt. Die Filtrate werden vereinigt. Das Lösungsmittel wird abgetrennt und der Rückstand wird über Kieselgel chromatographiert. Man erhält 163 mg (R)-*N*-BOC-Pyrrolidin-2-carbaldehyd ("4")

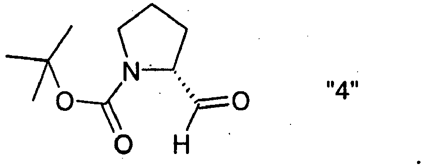

### Beispiel 2

### Herstellung von 6-[(R)-2-(4-Chlor-phenoxymethyl)-pyrrolidin-1-yl]-9H-purin ("A2")

2.1 Analog Beispiel 1.3 erhält man aus "2" und 4-Chlorphenol die Verbindung (R)-2-(4-Chlor-phenoxymethyl)-*N*-BOC-pyrrolidin ("7").
2.2 Durch Abspaltung der BOC-Gruppe erhält man aus "7" die Verbindung (R)-2-(4-Chlor-phenoxymethyl)-pyrrolidin ("8").
2.3 Analog Beispiel 1.5 erhält man durch Umsetzung von "8" mit 6-Chlorpurin die Verbindung "A2"; HPLC Retentionszeit 3,00 Minuten; Gradient P1; [M+H]⁺ 330.

### Beispiel 3

### Herstellung von 6-[(R)-2-(3,4-Difluor-phenoxymethyl)-pyrrolidin-1-yl]-9H-purin ("A3")

3.1 Analog Beispiel 1.3 erhält man aus "2" und 3,4-Difluor-phenol die Verbindung (R)-2-(3,4-Difluor-phenoxymethyl)-*N*-BOC-pyrrolidin ("9").
3.2 Durch Abspaltung der BOC-Gruppe erhält man aus "9" die Verbindung (R)-2-(3,4-Difluor-phenoxymethyl)-pyrrolidin ("10").
3.3 Analog Beispiel 1.5 erhält man durch Umsetzung von "10" mit 6-Chlorpurin die Verbindung "A3"; HPLC Retentionszeit 2,91 Minuten; Gradient P1; [M+H]⁺ 332.

### Analog Beispiel 1 werden die nachfolgenden Verbindungen erhalten

| Beispiel | Name | Retentionszeit [min] | Gradient HPLC | [M+H]⁺ |
|---|---|---|---|---|
| "A4" | 6-[(R)-2-(4-Methyl-phenoxymethyl)-pyrrolidin-1-yl]-9*H*-purin | 2,93 | P1 | 310 |
| "A5" | 6-[(R)-2-(Pyridin-2-yl-oxymethyl)-pyrrolidin-1-yl]-9*H*-purin | 2,35 | P1 | 297 |
| "A6" | 6-[(R)-2-(6-Methyl-pyridin-2-yl-oxymethyl)-pyrrolidin-1-yl]-9*H*-purin | 2,45 | P1 | 311 |
| "A7" | 6-[(R)-2-(2,5-Difluor-phenoxymethyl)-pyrrolidin-1-yl]-9*H-*purin | 2,87 | P1 | 332 |
| "A8" | 6-[(R)-2-(3,5-Difluor-phenoxymethyl)-pyrrolidin-1-yl]-9*H-*purin | 2,93 | P1 | 332 |
| "A9" | 6-[(R)-2-(2,4-Difluor-phenoxymethyl)-pyrrolidin-1-yl]-9*H-*purin | 2,85 | P1 | 332 |
| "A10" | 6-[(R)-2-(2-Fluor-phenoxymethyl)-pyrrolidin-1-yl]-9*H*-purin | 2,80 | P1 | 314 |
| "A11" | 6-[(R)-2-(3-Fluor-phenoxymethyl)-pyrrolidin-1-yl]-9*H*-purin | 2,87 | P1 | 314 |
| "A12" | 6-[(R)-2-(2,3-Difluor-phenoxymethyl)-pyrrolidin-1-yl]-9*H-*purin | 2,87 | P1 | 332 |
| "A13" | 6-[(R)-2-(4-Methoxy-phenoxymethyl)-pyrrolidin-1-yl]-9*H-*purin | 2,79 | P1 | 326 |
| "A14" | 6-[(R)-2-(4-Cyan-phenoxymethyl)-pyrrolidin-1-yl]-9*H*-purin | 2,75 | P1 | 321 |
| "A15" | 6-[(R)-2-(4-Fluor-phenoxymethyl)-pyrrolidin-1-yl]-9*H*-purin | 2,85 | P1 | 314 |

### Beispiel 4

### Herstellung von 6-[(R)-2-(2-Methoxy-phenoxymethyl)-pyrrolidin-1-yl]-9H-purin ("A16")

4.1 Eine Mischung von 75 mg "3", 58,99 µl 2-Methoxyphenol und 87,3 mg Cäsiumcarbonat in 1,5 ml DMF wird 16 Stunden bei 100° gerührt. Das Gemisch wird mit 10 ml Wasser verdünnt und dreimal mit Ethylacetat extrahiert. Die organischen Phasen werden vereinigt und es wird weiter wie üblich aufgearbeitet. Man erhält 63,7 mg (R)-2-(2-Methoxy-phenoxymethyl)-*N*-BOC-pyrrolidin ("11").
4.2 Durch Abspaltung der BOC-Gruppe erhält man aus "11" die Verbindung (R)-2-(2-Methoxy-phenoxymethyl)-pyrrolidin, Trifluoracetat ("12").
4.3 Analog Beispiel 1.5 erhält man durch Umsetzung von "12" mit 6-Chlorpurin die Verbindung "A16"; HPLC Retentionszeit 2,64 Minuten; Gradient P2; [M+H]⁺ 326.

### Analog werden die nachfolgenden Verbindungen erhalten

| Beispiel | Name | Retentionszeit [min] | Gradient HPLC | [M+H]⁺ |
|---|---|---|---|---|
| "A17" | 6-[(R)-2-(2-Ethyl-phenoxymethyl)-pyrrolidin-1-yl]-9*H*-purin | 2,94 | P2 | 324 |
| "A18" | 6-[(R)-2-(2-Methyl-phenoxymethyl)-pyrrolidin-1-yl]-9*H*-purin | 2,83 | P2 | 310 |
| "A19" | 6-[(R)-2-(Phenoxymethyl)-pyrrolidin-1-yl]-9*H*-purin | 2,71 | P2 | 296 |
| "A20" | 6-[(R)-2-(2-Chlor-phenoxymethyl)-pyrrolidin-1-yl]-9*H*-purin | 2,87 | P2 | 330 |
| "A21" | 6-[(R)-2-(2,3-Dichlor-phenoxymethyl)-pyrrolidin-1-yl]-9*H-*purin | 3,01 | P2 | 365 |
| "A22" | 6-[(R)-2-(2-Trifluormethyl-phenoxymethyl)-pyrrolidin-1-yl]-9*H-*purin | 2,95 | P2 | 364 |
| "A23" | 6-[(R)-2-(2,4-Dichlor-phenoxymethyl)-pyrrolidin-1-yl]-9*H-*purin | 3,03 | P2 | 365 |
| "A24" | 6-[(R)-2-(2-Chlor-5-methyl-phenoxymethyl)-pyrrolidin-1-yl]-9*H-*purin | 2,95 | P2 | 344 |
| "A25" | 6-[(R)-2-(2,6-Dichlor-phenoxymethyl)-pyrrolidin-1-yl]-9*H-*purin | 2,95 | P2 | 365 |
| "A26" | 6-[(R)-2-(2,5-Dichlor-phenoxymethyl)-pyrrolidin-1-yl]-9*H-*purin | 3,04 | P2 | 365 |
| "A27" | 6-[(R)-2-(2-chlor-4-fluor-phenoxymethyl)-pyrrolidin-1-yl]-9*H-*purin | 2,89 | P2 | 348 |
| "A28" | 6-[(R)-2-(2-chlor-4-methoxy-phenoxymethyl)-pyrrolidin-1-yl]-9*H-*purin | 2,82 | P2 | 360 |
| "A29" | 2-Chlor-6-[(R)-2-(2-chlor-phenoxymethyl)-pyrrolidin-1-yl]-9*H-*purin | 3,47 | P2 | 365 |
| "A30" | 6-[(R)-2-(4-Chlor-pyridin-3-yl-oxymethyl)-pyrrolidin-1-yl]-9*H*-purin | 2,56 | P2 | 348 |

### Beispiel 5

### Herstellung von 6-{(R)-2-[(2-Fluor-phenylamino)-methyl]-pyrrolidin-1-yl}-9H-purin ("A31")

5.1 Eine Mischung von 50 mg "4" und 26,67 µl 2-Fluoranilin in 1 ml Dichlormethan wird bei 0° mit 82,24 mg Natriumtriacteoxyborhydrid versetzt. Man läßt auf Raumtemperatur erwärmen und rührt 16 Stunden nach. Das Reaktionsgemisch wird über Kieselgel chromatographiert. Man erhält 30,9 mg (R)-2-[(2-Fluor-phenylamino)-methyl]-*N*-BOC-pyrrolidin ("13").
5.2 Durch Abspaltung der BOC-Gruppe erhält man aus "13" die Verbindung (R)-2-[(2-Fluor-phenylamino)-methyl]-pyrrolidin, Bistrifluoracetat ("14").
5.3 Analog Beispiel 1.5 erhält man durch Umsetzung von "14" mit 6-Chlorpurin die Verbindung "A31"; HPLC Retentionszeit 2,71 Minuten; Gradient P2; [M+H]⁺ 313.

### Analog erhält man die nachstehenden Verbindungen

6-{((R)-2-[(Phenylamino)-methyl]-pyrrolidin-1-yl}-9*H*-purin ("A32"); HPLC Retentionszeit 2,54 Minuten; Gradient P2; [M+H]⁺ 295;
6-{((R)-2-[(2-Chlor-phenylamino)-methyl]-pyrrolidin-1-yl}-9*H*-purin ("A33"), HPLC Retentionszeit 2,78 Minuten; Gradient P2; [M+H]⁺ 329.

### Beispiel 6

### Herstellung von 6-{(R)-2-[(2-Chlor-phenylamino)-carbonyl]-pyrrolidin-1-yl}-9H-purin ("A34")

6.1 In einer 8 ml Kunststoffhülse mit Fritte werden 150 mg BOC-D-Prolin und 696,87 mg Triphenylphosphin (polymer gebunden) vorgelegt, mit 4 ml Dichlormethan versetzt und 5 Minuten geschüttelt. Dann tropft man 139,75 µl Trichloracetonitril zu und schüttelt 4 Stunden bei Raumtemperatur weiter. Anschließend wird die Reaktionslösung über die Fritte direkt in eine zweite (12 ml) Kunststoffhülse filtriert, in der sich 73,48 µl 2-Chloranilin, 597,3 mg Morpholinomethyl-polystyrol und 1 ml Dichlormethan befinden. Man rührt 16 Stunden bei Raumtemperatur nach. Danach wird filtriert, das Filtrat wird eingeengt und der Rückstand wird über eine RP18 Säule chromatographiert. Nach üblicher Aufarbeitung erhält man 14,3 mg (R)-2-[(2-Chlor-phenylamino)-carbonyl]-*N*-BOC-pyrrolidin ("15").
6.2 Durch Abspaltung der BOC-Gruppe erhält man aus "15" die Verbindung (R)-2-[(2-Chlor-phenylamino)-carbonyl]-pyrrolidin, Trifluoracetat ("16").
6.3 Analog Beispiel 1.5 erhält man durch Umsetzung von "16" mit 6-Chlorpurin die Verbindung "A34"

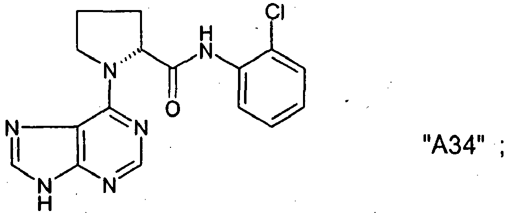

HPLC Retentionszeit 2,63 Minuten; Gradient P2; [M+H]⁺ 343.

### Analog erhält man die nachstehenden Verbindungen

6-{(R)-2-[(2-Fluor-phenylamino)-carbonyl]-pyrrolidin-1-yl}-9*H*-purin ("A35"); HPLC Retentionszeit 2,51 Minuten; Gradient P2; [M+H]⁺ 327.

### Beispiel 7

7.1 Zu einer Mischung von 75 mg (R)-2-(2-Chlor-phenoxymethyl)-pyrrolidin Trifluoracetat und 39,5 mg 2-Amino-6-chlor-9*H*-purin in 1 ml n-Butanol tropft man 93,84 µl Triethylamin. Das Reaktionsgemisch wird 2,5 Stunden bei 100° gerührt. Man kühlt ab, trennt den ausgefallenen Niederschlag ab und chromatographiert das Filtrat direkt über eine 12 g RP18-Säule. Die isolierten Produktfraktionen werden wie üblich aufgereinigt. Man erhält 45,4 mg 2-Amino-6-[(R)-2-(2-chlor-phenoxy-methyl)-pyrrolidin-1-yl]-9*H*-purin ("A36"); HPLC Retentionszeit 2,77 Minuten; Gradient P2; [M+H]⁺ 345.
7.2 35 mg "A36" werden unter Rühren bei Raumtemperatur in 50 µl Pyridin suspendiert, mit 132 µl HF/Pyridin versetzt und dann werden 16 µl tert.-Butylnitrit zugetropft.

Das Reaktionsgemisch wird mit einer Suspension von 530 mg CaCO₃ in 1 ml Wasser und 0,6 ml Methanol versetzt und 2 Stunden bei Raumtemperatur gerührt. Die Suspension wird abgesaugt und mit Ethylacetat gewaschen. Das Filtrat wird wie üblich aufgearbeitet. Nach Entfernung des Lösungsmittels wird der Rückstand über eine 4 g RP18 Kieselgelsäule chromatographiert. Die isolierten Fraktionen werden vereinigt und wie üblich aufgearbeitet. Man erhält 12,3 g 2-Fluor-6-[(R)-2-(2-chlor-phenoxy-methyl)-pyrrolidin-1-yl]-9*H*-purin ("A37"); HPLC Retentionszeit 2,51 Minuten; Gradient N; [M+H]⁺ 348.

### Beispiel 8

8.1 Eine Mischung von 100 mg "3", 50,3 µl 2-Fluorphenol, 116,6, mg Cäsiumcarbonat und 2 ml DMF wird 16 Stunden bei 100° gerührt.
   Man arbeitet wie üblich auf und erhält 49,5 mg (R)-2-(2-fluorphenoxymethyl)-*N*-BOC-pyrrolidin.
8.2 Durch Abspaltung der BOC-Gruppe erhält man daraus die Verbindung (R)-2-(2-Fluor-phenoxymethyl)-pyrrolidin, Trifluoracetat.
8.3 Zu einer Lösung von 28,9 mg (R)-2-(2-Fluor-phenoxymethyl)-pyrrolidin, Trifluoracetat, 16,2 mg 2,6-Dichlor-9*H*-purin und 1 ml n-Butanol tropft man 34,6 µl Triethylamin.
Das Reaktionsgemisch wird zur Aufreinigung direkt über eine 4 g RP18 Säule chromatographiert. Die isolierten Fraktionen werden vereinigt und wie üblich aufgearbeitet.
Man erhält 20,4 mg 2-Chlor-6-[(R)-2-(2-fluorphenoxymethyl)-pyrrolidin-1-yl]-9*H*-purin ("A38"); HPLC Retentionszeit 2,56 Minuten; Gradient P2; [M+H]⁺ 348.

### Beispiel 9

9.1 Durch Umsetzung von 1,0 g (R)-5-Oxo-pyrrolidin-2-carbonsäure-ethylester, 949,8 µl Benzylchloroformiat, 6,9 ml Bis-(trimethylsilyl)-lithiumamid (in THF) in 25 ml THF erhält man unter Standardbedingungen und Aufarbeitung 730 mg (R)-5-Oxo-*N*-benzyloxycarbonyl-pyrrolidin-2-carbonsäureethylester ("17").
9.2 123 mg Magnesiumspäne werden unter Argon in 1 ml THF vorgelegt und mit einigen Kristallen lod versetzt. Man wartet bis zur Entfärbung. Dann tropft man eine Lösung von 393,4 µl 4-Brom-1-buten in 11 ml THF so zu, daß eine Temperatur von 35 bis 40° gehalten wird. Man rührt noch 45 Minuten bei dieser Temperatur weiter. Anschließend tropft man 746,7 µl N,N,N',N'-Tetramethylethylendiamin zu und rührt 5 Minuten nach.
   Diese Lösung wird danach innerhalb von 10 Minuten zu einer auf -65 bis -70° gekühlten Lösung von 730 mg "17" in 13 ml THF zugetropft. Das Reaktionsgemisch wird noch 1,5 Stunden weitergerührt und dann mit 1,75 ml 2-Propanol versetzt.
   Man läßt das Reaktionsgemisch auf Raumtemperatur erwärmen und gibt alles in 15 ml einer 10 %igen H₃PO₄-Lösung. Man verdünnt mit Diethylether, arbeitet wie üblich auf und erhält 466 mg "18" 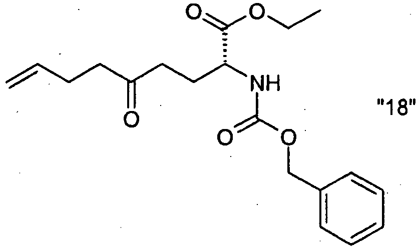
9.3 Zu einer Lösung von 632,8 mg Triphenylsilan in 2 ml Dichlormethan gibt man bei Raumtemperatur 610,5 µl Bortrifluorid-Diethylether-Komplex und rührt 10 Minuten nach. Diese Lösung tropft man dann zu einer auf -78° gekühlten Lösung von 469 mg "18" in 4 ml Dichlormethan.
   Man rührt noch 30 Minuten bei dieser Temperatur und anschließend 2 Stunden bei Raumtemperatur. Das Reaktionsgemisch wird auf -78° abgekühlt und wird dann in 30 ml gesättigte NaHCO₃-Lösung gegossen und 5 Minuten kräftig gerührt. Von der organischen Phase wird das Lösungsmittel abgetrennt. Der Rückstand wird über Kieselgel chromatographiert. Man erhält 396 mg (2R,5R)-5-But-3-enyl-*N-*benzyloxycarbonyl-pyrrolidin-2-carbonsäureethylester ("19") 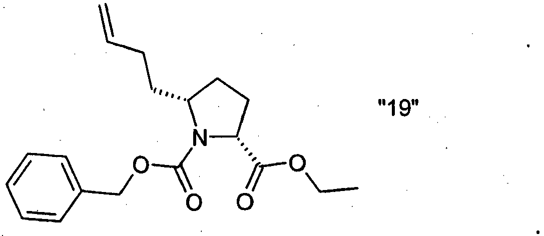
9.4 Zu einer Lösung von 396 mg "19" und 65,1 mg Natriumborhydrid in 5 ml Ethanol gibt man langsam eine Lösung von 63,6 mg Calciumchlorid in 3 ml Ethanol. Die Suspension wird 16 Stunden nachgerührt.
   Man arbeitet wie üblich auf und erhält 343 mg (2R,5R)-5-But-3-enyl-*N-*benzyloxycarbonyl-2-hydroxymethyl-pyrrolidin ("20").
9.5 Zu einer Lösung von 343 mg "20" in 5 ml Dichlormethan gibt man 302,3 µl Triethylamin und tropft dann 126,8 µl Methansulfonylchlorid zu.
   Man rührt 4 Stunden nach. Man verdünnt mit Dichlormethan, arbeitet wie üblich auf und erhält 405 mg "21" 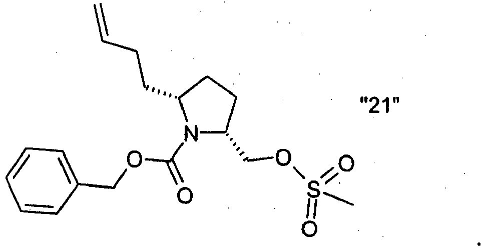
9.6 Analog Beispiel 4 erhält man durch Umsetzung von 150 mg "21" mit 77,5 µl 2-Chlorphenol 167 mg "22" 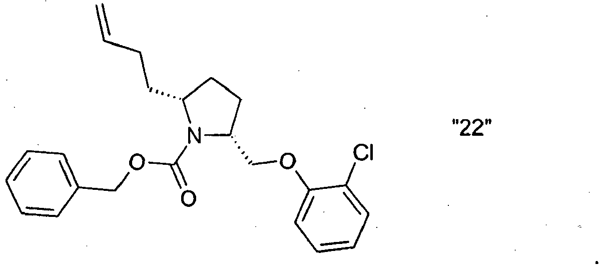
9.7 Zu einer Lösung von 167 mg "22" in 1,0 ml Eisessig gibt man unter Argonatmosphäre 132 µl Bromwasserstoff (33 %ige Lösung in Essigsäure) und rührt 3,5 Stunden nach. Nach Entfernen des Lösungsmittels wird der Rückstand über einer RP18 Kieselgelsäule chromatographiert. Die isolierten Fraktionen werden vereinigt und die Lösungsmittel werden entfernt. Der wässrige Rückstand wird mit 2N NaOH basisch gestellt und 3x mit Ethylacetat extrahiert. Die organischen Phasen werden vereinigt, mit Wasser und gesättigter Citronensäurelösung gewaschen und über Na₂SO₄-Lösung getrocknet. Man filtriert, entfernt das Lösungsmittel und erhält 59,2 mg (2R,5R)-5-But-3-enyl- 2-(2-chlor-phenoxymethyl)-pyrrolidin ("23").
9.8 Zu einer Mischung von 59,2 mg "23" und 35,8 mg 6-Chlorpurin in 1 ml Butanol tropft man 45,8 µl Triethylamin. Es wird 1,5 Stunden bei 100° und 16 Stunden bei Raumtemperatur nachgerührt. Das Gemisch wird direkt über eine 12 g RP18 Säule chromatographiert. Es wird analog 9.7 weiter aufgearbeitet.
   Man erhält 66 mg 6-[(2R,5R)-2-But-3-enyl-5-(2-chlor-phenoxymethyl)-pyrrolidin-1-yl]-9*H*-purin ("A39"), HPLC Retentionszeit 2,43 Minuten;
   Gradient N; [M+H]⁺ 384.
9.9 Eine Lösung von 64 mg "A39", 46,8 mg 4-Methylmorpholin-N-oxid und 4 mg Osmiumtetroxid in 2,5 ml Aceton und 0,5 ml Wasser wird unter Lichtausschluß 2 Stunden gerührt. Man gibt 42 mg Natriumsulfit zu und rührt 1 Stunde weiter. Das Reaktionsgemisch wird mit Wasser und Ethylacetat verdünnt und geschüttelt. Man filtriert über Kieselgur. Die wässrige Phase wird abgetrennt. Aus der organischen Phase erhält man 50 mg 4-[(2S,5R)-5-(2-Chlor-phenoxymethyl)-1-(9*H*-purin-6-yl)-pyrrolidin-2-yl]-butan-1,2-diol ("A40"), HPLC Retentionszeit 1,65/1,72 Minuten; Gradient N; [M+H]⁺ 418; 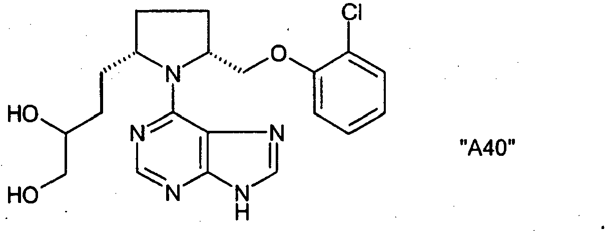
9.10 Durch Umsetzung von 55 mg "A40" mit 56,3 mg Natriummetaperiodat erhält man unter Standardbedingungen 16,6 mg "A40a" 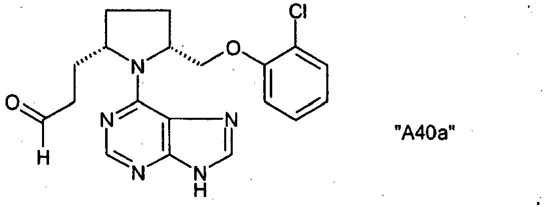
9.11 Aus 16,6 mg "A40a" erhält man durch Reduktion mit 1,48 mg Natriumborhydrid unter Standardbedingungen 16,4 mg 3-[(2S,5R)-5-(2-Chlor-phenoxymethyl)-1-(9*H*-purin-6-yl)-pyrrolidin-2-yl]-propan-1-ol ("A41"), HPLC Retentionszeit 1,96 Minuten; Gradient N; [M+H]⁺ 388;

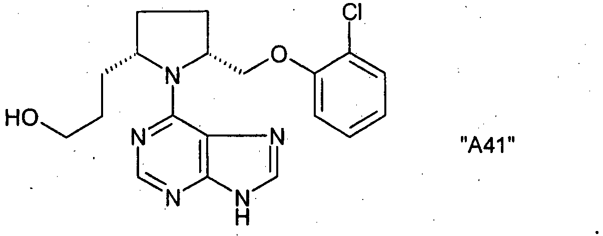

### Beispiel 10

### Analog Beispiel 6 werden die nachfolgenden Verbindungen erhalten

| Beispiel | Name / Struktur | Retentionszeit [min] | Gradient HPLC | [M+H]⁺ |
|---|---|---|---|---|
| "A42" | 6-{(R)-2-[(2-Fluor-benzylamino)-carbonyl]-pyrrolidin-1-yl}-9*H*-purin | 2,60 | P1 | 341 |
| | 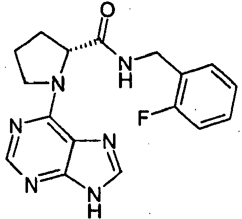 | | | |
| "A43" | 6-{(R)-2-[(4-Fluor-benzylamino)-carbonyl]-pyrrolidin-1-yl}-9*H*-purin | 2,61 | P1 | 341 |

### Beispiel 11

### Analog Beispiel 1 werden die nachfolgenden Verbindungen erhalten

| Beispiel | Name / Struktur | Retentionszeit [min] | Gradient HPLC | [M+H]⁺ |
|---|---|---|---|---|
| "A44" | 6-[(S)-2-(2-Chlor-phenoxymethyl)-pyrrolidin-1-yl]-9*H*-purin | 2,83 | P2 | 330 |
| "A45" | 6-[(S)-2-(Phenoxymethyl)-pyrrolidin-1-yl]-9*H*-purin | 1,71 | N | 296 |
| "A46" | 6-[(S)-2-(2-Methyl-phenoxymethyl)-pyrrolidin-1-yl]-9*H*-purin | 1,87 | N | 310 |
| "A47" | 2-Fluor-6-[(R)-2-(2-fluor-phenoxymethyl)-pyrrolidin-1-yl]-9*H-*purin | 3,21 | P2 | 332 |
| "A52" | 6-[(R)-2-(2-Brom-phenoxymethyl)-pyrrolidin-1-yl]-9*H*-purin | 1,89 | N | 375 |
| "A57" | 2-Methyl-6-[(R)-2-(2-fluor phenoxymethyl)-pyrrolidin-1-yl]-9*H-*purin | 2,67 | P2 | 328 |
| "A58" | 2-Methyl-6-[(R)-2-(2-chlor-phenoxymethyl)-pyrrolidin-1-yl]-9*H-*purin | 2,75 | P2 | 344 |
| "A59" | 6-[(R)-2-(2-Chlor-phenoxymethyl)-pyrrolidin-1-yl]-9*H*-purin | 2,17 | P2 | 331 |

### Beispiel 12

### Herstellung von 2-Chlor-6-[(2R,4S)-2-(2-Chlor-phenoxymethyl)-4-prop-2-ynyloxy-pyrrolidin-1-yl]-9H-purin ("A48")

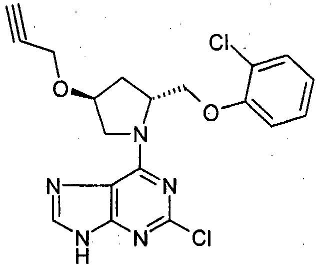

12.1 20 g (2R,4S)-4-Hydroxy-prolin-ethylester werden unter Argon in 200 ml Dichlormethan suspendiert und mit 42,5 ml Triethylamin versetzt.Dann gibt man 1,25 g 4-(Dimethylamino)-pyridin zu und tropft eine Lösung von 25,04 g Di-tert.-butyldicarbonat in 150 ml Dichlormethan zu. Man rührt 16 Stunden nach und arbeitet wie üblich auf. Man erhält 28,01 g 1-tert.-Butyloxycarbonyl-(2R,4S)-4-hydroxy-prolin-ethylester als Öl.
12.2 Unter Argonatmosphäre gibt man 120 ml DMF zu 28,01 g 1-tert.-Butyloxycarbonyl-(2R,4S)-4-Hydroxyprolin-ethylester, 30,28 g tert.-Butyldimethylchlorsilan und 16,41 g Imidazol. Man rührt 2 Stunden nach. Man versetzt mit MTB-Ether und arbeitet wie üblich auf. Der Rückstand wird über Kieselgel chromatographiert. Man erhält 40,4 g 1-tert.-Butyloxycarbonyl-(2R,4S)-4-(tert.-butyldimethyl-silyloxy)-prolin-ethylester als Öl.
12.3 Durch Reduktion mit Natriumborhydrid in THF erhält man unter Standardbedingungen aus 20 g 1-tert.-Butyloxycarbonyl-(2R,4S)-4-(tert.-butyldimethyl-silyloxy)-prolin-ethylester 16,4 g der Verbindung 1-tert.-Butyloxycarbonyl-(2R,4S)-4-(tert.-butyldimethyl-silyloxy)-prolinol 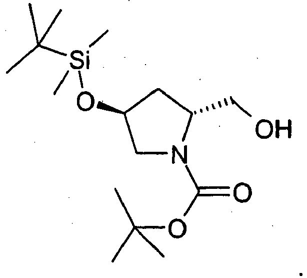
12.4 Durch Umsetzung von 8,54 g 1-tert.-Butyloxycarbonyl-(2R,4S)-4-(tert.-butyldimethyl-silyloxy)-prolinol mit 2,59 ml Methansulfonylchlorid in 85 ml Dichlormethan und 5,3 ml Triethylamin erhält man unter Standardbedingungen 9,48 g der Verbindung 1-tert.-Butyloxycarbonyl-(2R,4S)-2-(methylsulfonyloxymethyl)-4-(tert.-butyldimethyl-silyloxy)-pyrrolidin.
12.5 Durch Umsetzung von 1,5 g 1-tert.-Butyloxycarbonyl-(2R,4S)-2-(methylsulfonyloxymethyl)-4-(tert.-butyldimethyl-siyloxy)-pyrrolidin mit 560 µl 2-Chlorphenol und 1,19 g Cäsiumcarbonat in 15 ml DMF erhält man unter Standardbedingungen 751 mg (2R,4S)-1-tert.-Butyloxycarbonyl-2-(2-chlor-phenoxymethyl)- 4-(tert.-butyldimethyl-silyloxy)-pyrrolidin. Als Nebenprodukt entsteht 223 mg des silylabgespaltenen Produkts (2R,4S)-1-tert.-Butyloxycarbonyl-2-(2-chlor-phenoxymethyl)-4-hydroxy-pyrrolidin.
12.6 Die Abspaltung der Silylgruppe erfolgt unter Standardbedingungen aus (2R,4S)- 1-tert.-Butyloxycarbonyl-2-(2-chlor-phenoxymethyl)-4-(tert.-butyldimethyl-silyloxy)-pyrrolidin mit Tetra-n-butylammoniumfluorid in THF. Man erhält (2R,4S)-2-(2-Chlor-phenoxymethyl)-4-hydroxy-pyrrolidin.
12.7 Zu einer Lösung von 310 mg (2R,4S)-1-tert.-Butyloxycarbonyl-2-(2-chlor-phenoxymethyl)-4-hydroxy-pyrrolidinin 3 ml DMF gibt man zuerst 1,16 g Propagylbromid und unter N₂-Atmosphäre 34,04 mg NaH. Anschließend gibt man 219,15 mg Silberoxid zuund rührt 18 Stunden nach. Man arbeitet wie üblich auf und erhält nach Chromatographie über Kieselgel 200 mg (2R,4S)-1-tert.-Butyloxycarbonyl-2-(2-chlor-phenoxymethyl)-4-prop-2-ynyloxy-pyrrolidin.
12.8 Die Abspaltung der BOC-Gruppe aus 200 mg (2R,4S)-1-tert.-Butyloxycarbonyl-2-(2-chlor-phenoxymethyl)-4-prop-2-ynyloxy-pyrrolidin erfolgt mit TFA in Dichlormethan unter Standardbedingungen. Man erhält 285 mg (2R,4S)-2-(2-Chlor-phenoxymethyl)-4-prop-2-ynyloxy-pyrrolidin, Di-trifluoracetat.
12.9 Zu einer Lösung von 50 mg (2R,4S)-2-(2-Chlor-phenoxymethyl)-4-prop-2-ynyloxy-pyrrolidin-di-trifluoracetat und 18,54 mg 2,6-Dichlor-9H-purin in 1 ml n-Butanol gibt man 39,83 µl Triethylamin. Das Gemisch wird 45 Minuten bei 100° gerührt. Man kühlt ab, engt in,der Vakuumzentrifuge auf ca. 20% ein und reinigt chromatographisch über eine 12 g RP18-Säule.
   Eluent: Wasser + 0,1% HCOOH / Acetonitril + 0,1% HCOOH 99:1 - 0:100 in 18 Minuten.
   Die Fraktionen, die das Produkt enthalten werden vereinigt. Die Lösungsmittel werden entfernt. Man stellt mit 2N NaOH auf pH 10-11 und arbeitet wie üblich auf. Man erhält 36,1 g "A48"; Retentionszeit 3,37 min; Gradient HPLC P2; [M+H]⁺ 418.

### Analog erhält man die Verbindungen

6-[((2R,4S)-2-(2-Chlor-phenoxymethyl)-4-prop-2-ynyloxy-pyrrolidin-1-yl]-9*H*-purin ("A49"); Retentionszeit 2,95 min; Gradient HPLC P2; [M+H]⁺ 384;
2-Chlor-6-[(2R,4S)-2-(2-chlor-phenoxymethyl)-4-allyloxy-pyrrolidin-1-yl]-9*H*-purin ("A50"); Retentionszeit 3,51 min; Gradient HPLC P2; [M+H]⁺ 421;
6-[((2R,4S)-2-(2-Chlor-phenoxymethyl)-4-allyloxy-pyrrolidin-1-yl]-9*H-*purin ("A51 "); Retentionszeit 3,01 min; Gradient HPLC P2; [M+H]⁺ 386;
2-Chlor-6-[(2R,4S)-2-phenoxymethyl-4-hydroxy-pyrrolidin-1-yl]-9*H-*purin ("A53"); Retentionszeit 2,92 min; Gradient HPLC P2; [M+H]⁺ 346;
2-Chlor-6-[(2R,4S)-2-(2-fluor-phenoxymethyl)-4-hydroxy-pyrrolidin-1-yl]-9*H*-purin ("A54"); Retentionszeit 2,93 min; Gradient HPLC P2; [M+H]⁺ 365;
2-Chlor-6-[(2R,4S)-2-(2-fluor-phenoxymethyl)-4-prop-2-ynyloxy-pyrrolidin-1-yl]-9*H*-purin ("A55"); Retentionszeit 3,29 min; Gradient HPLC P2; [M+H]⁺ 402;
2-Chlor-6-[(2R,4S)-2-phenoxymethyl-4-prop-2-ynyloxy-pyrrolidin-1-yl]-9*H*-purin ("A56"); Retentionszeit 3,28 min; Gradient HPLC P2; [M+H]⁺ 384.

### DEI-MS fragment interpretation

### Compound "A29"

| fragment | formula | mass/charge | proposed structure |
|---|---|---|---|
| M^{┐+·} | C₁₆H₁₅Cl₂N₅O | 363 | 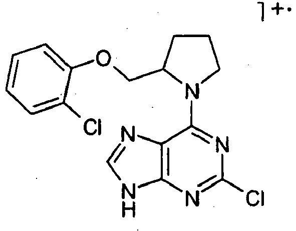 |
| 236^{┐+} | C₁₀H₁₁ClN₅ | 236 | 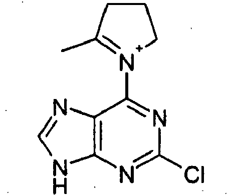 |
| 222^{┐+} | C₉H₉ClN₅ | 222 | 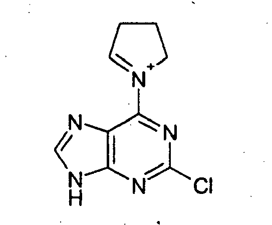 |
| 153^{┐+} | C₅H₂ClN₄ | 153 | 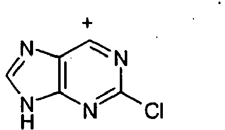 |

### Compound "A20"

| fragment | formula | mass/charge | proposed structure |
|---|---|---|---|
| M^{┐+·} | C₁₆H₁₆ClN₅O | 329 | 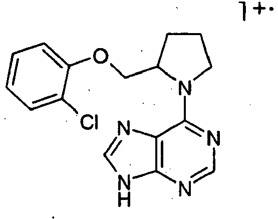 |
| 202^{┐+} | C₁₀H₁₂N₅ | 202 | 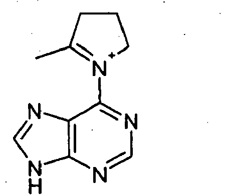 |
| 188^{┐+} | C₉H₁₀N₅ | 188 | 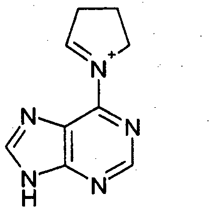 |
| 119^{┐+} | C₅H₃N₄ | 119 | 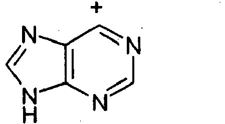 |

### Compound "A38"

| fragment | formula | mass/charge | proposed structure |
|---|---|---|---|
| M^{┐+·} | C₁₆H₁₅ClFN₅O | 347 | 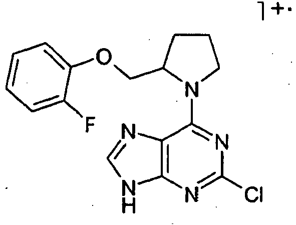 |
| 236^{┐+} | C₁₀H₁₁ClN₅ | 236 | 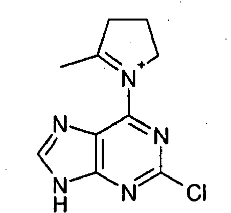 |
| 222^{┐+} | C₉H₉ClN₅ | 222 | 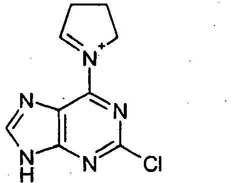 |
| 153^{┐+} | C₅H₂ClN₄ | 153 | 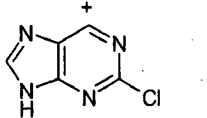 |

### Compound "A10"

| fragment | formula | mass/charge | proposed structure |
|---|---|---|---|
| M^{┐+·} | C₁₆H₁₆FN₅O | 313 | 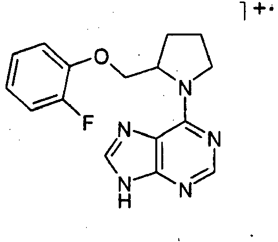 |
| 202^{┐+} | C₁₀H₁₂N₅ | 202 | 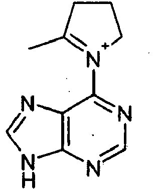 |
| 188^{┐+} | C₉H₁₀N₅ | 188 | 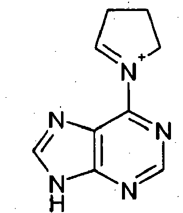 |
| 119^{┐+} | C₅H₃N₄ | 119 | 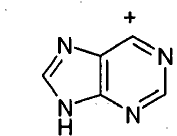 |

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄· 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 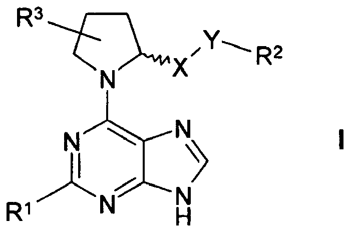 worin
X CH₂ oder CO,
Y O S, NH(CH₂)ₙ oder CH₂,
R¹ H,
R² Ar oder Het,
R³ H, OH, (CH₂)ₙOH, oder (CH₂)ₚCH(OH)(CH₂)ₚOH,
A, A' jeweils unabhängig voneinander unverzweigtes oder verzweigtes Alkyl mit 1-10 C-Atomen, worin 1-5 H-Atome durch F, Cl und/oder Br ersetzt sein können,
Alk oder cyclisches Alkyl mit 3-7 C-Atomen,
A und A' zusammen auch eine Alkylenkette mit 2, 3, 4, 5 oder 6 C-Atomen, worin eine oder zwei CH₂-Gruppen durch O, S, SO, SO₂, NH, NA und/oder N-COOA ersetzt sein können,
Alk Alkenyl mit 2-6 C-Atomen,
Ar unsubstituiertes oder ein-, zwei-, drei-, vier- oder fünffach durch A, OA, OH, SH, SA, Hal, NO₂ CN, (CH₂)ₙAr', (CH₂)ₙCOOH, (CH₂)ₙCOOA, CHO, COA, SO₂A, CONH₂, SO₂NH₂, CONHA, CONAA', SO₂NHA SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA',NACONAA', NHCO(CH₂)ₙNH₂ und/oder -O-(CH₂)ₒ-Het¹ substituiertes Phenyl, Naphthyl oder Biphenyl,
Ar' unsubstituiertes oder ein-, zwei- oder dreifach durch A, OA, OH, SH, SA, Hal, NO₂, CN, (CH₂)ₙPhenyl, (CH₂)ₙCOOH, (CH₂)ₙCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA' und/oder NACONAA' substituiertes Phenyl, Naphthyl oder Biphenyl,
Het einen ein- oder zweikernigen gesättigten, ungesättigten oder aromatischen Heterocyclus mit 1 bis 4 N-, O-und/oder S-Atomen, der ein-, zwei- oder dreifach durch A, OA, OH, SH, SA, Hal, NO₂, CN, (CH₂)ₙAr', (CH₂)ₙCOOH, (CH₂)ₙCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂A, NHCONH₂, NACONH₂, NHCONHA, NACONHA, NHCONAA', NACONAA', SO₂A, =S, =NH, =NA und/oder =O (Carbonylsauerstoff) substituiert sein kann,
Het¹ einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,
Hal F, Cl, Br oder I,
m 2, 3, 4, 5 oder 6
n 0, 1, 2, 3 oder 4,
o 1 oder 2,
p 1, 2, 3 oder 4,
bedeuten,
sowie ihre pharmazeutisch verwendbaren Salze, Solvate, Tautomere und Stereoisomere, einschließlich deren Mischungen in allen Verhältnissen.

2. Verbindungen nach Anspruch 1 der Formel I, worin
Y O oder NH(CH₂)ₙ bedeutet,
sowie ihre pharmazeutisch verwendbaren Salze, Solvate, Tautomere und Stereoisomere, einschließlich deren Mischungen in allen Verhältnissen.

3. Verbindungen nach Anspruch 1 oder 2, worin
R³ H, OH, oder (CH₂)ₚCH(OH)(CH₂)ₚOH bedeutet,
sowie ihre pharmazeutisch verwendbaren Salze, Solvate, Tautomere und Stereoisomere, einschließlich deren Mischungen in allen Verhältnissen.

4. Verbindungen nach einem oder mehreren der Ansprüche 1-3, worin
Ar unsubstituiertes oder ein-, zwei-, drei-, vier- oder fünffach durch A, Hal, OA, OH oder CN substituiertes Phenyl,
bedeutet,
sowie ihre pharmazeutisch verwendbaren Salze, Solvate, Tautomere und Stereoisomere, einschließlich deren Mischungen in allen Verhältnissen.

5. Verbindungen nach einem oder mehreren der Ansprüche 1-4, worin
Het einen einkernigen aromatischen Heterocyclus mit 1 bis 3 N-Atomen, der ein-, zwei- oder dreifach durch A, OA und/oder Hal substituiert sein kann,
bedeutet,
sowie ihre pharmazeutisch verwendbaren Salze, Solvate, Tautomere und Stereoisomere, einschließlich deren Mischungen in allen Verhältnissen.

6. Verbindungen nach einem oder mehreren der Ansprüche 1-12, worin
A unverzweigtes oder verzweigtes Alkyl mit 1-6 C-Atomen, worin 1-5 H-Atome durch F und/oder Cl ersetzt sein können, oder Alk,
bedeutet,
sowie ihre pharmazeutisch verwendbaren Salze, Solvate, Tautomere und Stereoisomere, einschließlich deren Mischungen in allen Verhältnissen.

7. Verbindungen nach einem oder mehreren der Ansprüche 1-6, worin
X CH₂ oder CO,
Y O oder NH(CH₂)ₙ,
R¹ H,
R² Ar oder Het,
R³ H, OH, oder (CH₂)ₚCH(OH)(CH₂)ₚOH,
A unverzweigtes oder verzweigtes Alkyl mit 1-6 C-Atomen, worin 1-5 H-Atome durch F und/oder Cl ersetzt sein können, oder Alk,
Alk Alkenyl mit 2-6 C-Atomen,
Ar unsubstituiertes oder ein-, zwei-, drei-, vier- oder fünffach durch A, Hal, OA, OH oder CN substituiertes Phenyl,
Het einen einkernigen aromatischen Heterocyclus mit 1 bis 3 N-Atomen, der ein-, zwei- oder dreifach durch A, OA und/oder Hal substituiert sein kann,
Hal F, Cl, Br oder I,
p 1, 2, 3 oder 4,
n 0, 1 oder 2
bedeuten,
sowie ihre pharmazeutisch verwendbaren Salze, Solvate, Tautomere und Stereoisomere, einschließlich deren Mischungen in allen Verhältnissen.

8. Verbindungen nach Anspruch 1 ausgewählt aus der Gruppe
| Beispiel | Name |
|---|---|
| "A1" | 6-[(R)-2-(5-Fluor-2-methoxy-phenoxymethyl)-pyrrolidin-1-yl]-9*H*-purin |
| "A2" | 6-[(R)-2-(4-Chlor-phenoxymethyl)-pyrrolidin-1-yl]-9*H-*purin |
| "A3" | 6-[(R)-2-(3,4-Difluor-phenoxymethyl)-pyrrolidin-1-yl]-9*H-*purin |
| "A4" | 6-[(R)-2-(4-Methyl-phenoxymethyl)-pyrrolidin-1-yl]-9*H-*purin |
| "A5" | 6-[(R)-2-(Pyridin-2-yl-oxymethyl)-pyrrolidin-1-yl]-9*H*-purin |
| "A6" | 6-[(R)-2-(6-Methyl-pyridin-2-yl-oxymethyl)-pyrrolidin-1-yl]-9*H*-purin |
| "A7" | 6-[(R)-2-(2,5-Difluor-phenoxymethyl)-pyrrolidin-1-yl]-9*H-*purin |
| "A8" | 6-[(R)-2-(3,5-Difluor-phenoxymethyl)-pyrrolidin-1-yl]-9*H-*purin |
| "A9" | 6-[(R)-2-(2,4-Difluor-phenoxymethyl)-pyrrolidin-1-yl]-9*H-*purin |
| "A10" | 6-[(R)-2-(2-Fluor-phenoxymethyl)-pyrrolidin-1-yl]-9*H-*purin |
| "A11" | 6-[(R)-2-(3-Fluor-phenoxymethyl)-pyrrolidin-1-yl]-9*H-*purin |
| "A12" | 6-[(R)-2-(2,3-Difluor-phenoxymethyl)-pyrrolidin-1-yl]-9*H-*purin |
| "A13" | 6-[(R)-2-(4-Methoxy-phenoxymethyl)-pyrrolidin-1-yl]-9*H-*purin |
| "A14" | 6-[(R)-2-(4-Cyan-phenoxymethyl)-pyrrolidin-1-yl]-9*H-*purin |
| "A15" | 6-[(R)-2-(4-Fluor-phenoxymethyl)-pyrrolidin-1-yl]-9*H-*purin |
| "A16" | 6-[(R)-2-(2-Methoxy-phenoxymethyl)-pyrrolidin-1-yl]-9*H-*purin |
| "A17" | 6-[(R)-2-(2-Ethyl-phenoxymethyl)-pyrrolidin-1-yl]-9*H-*purin |
| "A18" | 6-[(R)-2-(2-Methyl-phenoxymethyl)-pyrrolidin-1-yl]-9*H-*purin |
| "A19" | 6-[(R)-2-(Phenoxymethyl)-pyrrolidin-1-yl]-9*H*-purin |
| "A20" | 6-[(R)-2-(2-Chlor-phenoxymethyl)-pyrrolidin-1-yl]-9*H-*purin |
| "A21" | 6-[(R)-2-(2,3-Dichlor-phenoxymethyl)-pyrrolidin-1-yl]-9*H-*purin |
| "A22" | 6-[(R)-2-(2-Trifluormethyl-phenoxymethyl)-pyrrolidin-1-yl]-9*H*-purin |
| "A23" | 6-[(R)-2-(2,4-Dichlor-phenoxymethyl)-pyrrolidin-1-yl]-9*H-*purin |
| "A24" | 6-[(R)-2-(2-Chlor-5-methyl-phenoxymethyl)-pyrrolidin-1-yl]-9*H*-purin |
| "A25" | 6-[(R)-2-(2,6-Dichlor-phenoxymethyl)-pyrrolidin-1-yl]-9*H-*purin |
| "A26" | 6-[(R)-2-(2,5-Dichlor-phenoxymethyl)-pyrrolidin-1-yl]-9*H-*purin |
| "A27" | 6-[(R)-2-(2-chlor-4-fluor-phenoxymethyl)-pyrrolidin-1-yl]-9*H*-purin |
| "A28" | 6-[(R)-2-(2-chlor-4-methoxy-phenoxymethyl)-pyrrolidin-1-yl]-9*H*-purin |
| "A29" | 2-Chlor-6-[(R)-2-(2-chlor-phenoxymethyl)-pyrrolidin-1-yl]-9*H*-purin |
| "A30" | 6-[(R)-2-(4-Chlor-pyridin-3-yl-oxymethyl)-pyrrolidin-1-yl]-9*H*-purin |
| "A31" | 6-{(R)-2-[(2-Fluor-phenylamino)-methyl]-pyrrolidin-1-yl}-9*H*-purin |
| "A32" | 6-{(R)-2-[(Phenylamino)-methyl]-pyrrolidin-1-yl}-9*H*-purin |
| "A33" | 6-{(R)-2-[(2-Chlor-phenylamino)-methyl]-pyrrolidin-1-yl}-9*H*-purin |
| "A34" | 6-{(R)-2-[(2-Chlor-phenylamino)-carbonyl]-pyrrolidin-1-yl}-9*H*-purin |
| "A35" | 6-{(R)-2-[(2-Fluor-phenylamino)-carbonyl]-pyrrolidin-1-yl}-9*H*-purin |
| "A36" | 2-Amino-6-[(R)-2-(2-chlor-phenoxy-methyl)-pyrrolidin-1-yl]-9*H*-purin |
| "A37" | 2-Fluor-6-[(R)-2-(2-chlor-phenoxy-methyl)-pyrrolidin-1-yl]-9*H*-purin |
| "A38" | 2-Chlor-6-[(R)-2-(2-fluorphenoxymethyl)-pyrrolidin-1-yl]-9*H*-purin |
| "A40" | 4-[(2S,5R)-5-(2-Chlor-phenoxymethyl)-1-(9*H*-purin-6-yl)-pyrrolidin-2-yl]-butan-1,2-diol |
| "A41" | 3-[(2S,5R)-5-(2-Chlor-phenoxymethyl)-1-(9*H*-purin-6-yl)-pyrrolidin-2-yl]-propan-1-ol |
| "A42" | 6-{(R)-2-[(2-Fluor-benzylamino)-carbonyl]-pyrrolidin-1-yl}-9*H*-purin |
| | 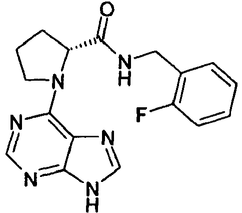 |
| "A43" | 6-{(R)-2-[(4-Fluor-benzylamino)-carbonyl]-pyrrolidin-1-yl}-9*H*-purin |
| "A44" | 6-[(S)-2-(2-Chlor-phenoxymethyl)-pyrrolidin-1-yl]-9*H-*purin |
| "A45" | 6-[(S)-2-(Phenoxymethyl)-pyrrolidin-1-yl]-9*H*-purin |
| "A46" | 6-[(S)-2-(2-Methyl-phenoxymethyl)-pyrrolidin-1-yl]-9*H-*purin |
| "A47" | 2-Fluor-6-[(R)-2-(2-fluor-phenoxymethyl)-pyrrolidin-1-yl]-9*H*-purin |
| "A48" | 2-Chlor-6-((2R,4S)-2-(2-Chlor-phenoxymethyl)-4-prop-2-ynyloxy-pyrrolidin-1-yl)-9*H*-purin |
| "A49" | 6-[(2R,4S)-2-(2-Chlor-phenoxymethyl)-4-prop-2-ynyloxy-pyrrolidin-1-yl]-9*H*-purin |
| "A50" | 2-Chlor-6-[(2R,4S)-2-(2-chlor-phenoxymethyl)-4-allyloxy-pyrrolidin-1-yl]-9*H*-purin |
| "A51" | 6-[(2R,4S)-2-(2-Chlor-phenoxymethyl)-4-allyloxy-pyrrolidin-1-yl]-9*H*-purin |
| "A52" | 6-[(R)-2-(2-Brom-phenoxymethyl)-pyrrolidin-1-yl]-9*H-*purin |
| "A53" | 2-Chlor-6-[(2R,4S)-2-phenoxymethyl-4-hydroxy-pyrrolidin-1-yl]-9*H*-purin |
| "A54" | 2-Chlor-6-[(2R,45)-2-(2-fluor-phenoxymethyl)-4-hydroxy-pyrrolidin-1-yl]-9*H*-purin |
| "A55" | 2-Chlor-6-[(2R,4S)-2-(2-fluor-phenoxymethyl)-4-prop-2-ynyloxy-pyrrolidin-1-yl]-9*H*-purin |
| "A56" | 2-Chlor-6-[(2R,4S)-2-phenoxymethyl-4-prop-2-ynyloxy-pyrrolidin-1-yl]-9*H*-purin |
| "A57" | 2-Methyl-6-[(R)-2-(2-fluor-phenoxymethyl)-pyrrolidin-1-yl]-9*H*-purin |
| "A58" | 2-Methyl-6-[(R)-2-(2-chlor-phenoxymethyl)-pyrrolidin-1-yl]-9*H*-purin |
| "A59" | 6-[(R)-2-(2-Chlor-phenoxymethyl)-pyrrolidin-1-yl]-9*H-*purin |
sowie ihre pharmazeutisch verwendbaren Salze, Solvate, Tautomere und Stereoisomere, einschließlich deren Mischungen in allen Verhältnissen.

9. Verfahren zur Herstellung von Verbindungen der Formel I nach den Ansprüchen 1-8 sowie ihrer pharmazeutisch verwendbaren Solvate, Salze, Tautomere und Stereoisomere, **dadurch gekennzeichnet, daß** man
eine Verbindung der Formel II 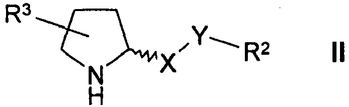 worin
X, Y, R² und R³ die in Anspruch 1 angegebenen Bedeutungen haben,
mit
einer Verbindung der Formel III 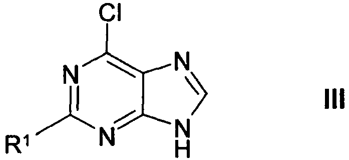
worin R¹ die in Anspruch 1 angegebene Bedeutung hat,
umsetzt,
und/oder eine Base oder Säure der Formel I in eines ihrer Salze umwandelt.

10. Arzneimittel, enthaltend mindestens eine Verbindung der Formel I und/oder ihre pharmazeutisch verwendbaren Salze, Solvate, Tautomere und Stereoisomere, einschließlich deren Mischungen in allen Verhältnissen, sowie gegebenenfalls Träger- und/oder Hilfsstoffe.

11. Verwendung von Verbindungen der Formel I sowie ihrer pharmazeutisch verwendbaren Salze, Solvate, Tautomere und Stereoisomere, einschließlich deren Mischungen in allen Verhältnissen, zur Herstellung eines Arzneimittels zur Behandlung und/oder Prophylaxe von Krankheiten, bei denen die Hemmung, Regulierung und/oder Modulation von HSP90 eine Rolle spielt.

12. Verwendung nach Anspruch 11 von Verbindungen der Formel I sowie ihrer pharmazeutisch verwendbaren Salze, Solvate, Tautomere und Stereoisomere, einschließlich deren Mischungen in allen Verhältnissen, zur Herstellung eines Arzneimittels zur Behandlung oder Vorbeugung von Tumorerkrankungen, viralen Erkrankungen, zur Immunsuppression bei Transplantationen, entzündungsbedingten Erkrankungen, Zystische Fibrose, Erkrankungen im Zusammenhang mit Angiogenese, infektiösen Erkrankungen, Autoimmunerkrankungen, Ischämie, fibrogenetischen Erkrankungen,
zur Förderung der Nervenregeneration,
zur Hemmung des Wachstums von Krebs, Tumorzellen und Tumormetastasen,
zum Schutz normaler Zellen gegen Toxizität, die durch Chemotherapie verursacht ist,
zur Behandlung von Krankheiten, wobei Proteinfehlfaltung oder Aggregation ein Hauptkausalfaktor ist.

13. Verwendung nach Anspruch 12, wobei es sich bei den Tumorerkrankungen um Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiosarkom, Rhabdomyosarkom, Kolonkarzinom, Pankreaskrebs, Brustkrebs, Ovarkrebs, Prostatakrebs, Plattenzellkarzinom, Basalzellkarzinom, Adenokarzinom, Schweißdrüsenkarzinom, Talgdrüsenkarzinom, Papillarkarzinom, Papillaradenokarzinomen, Cystadenokarzinomen, Knochenmarkkarzinom, bronchogenem Karzinom, Nierenzellkarzinom, Hepatom, Gallengangkarzinom, Chorionkarzinom, Seminom, embryonalem Karzinom, Wilms-Tumor, Cervix-Krebs, Hodentumor, Lungenkarzinom, kleinzelligem Lungenkarzinom, Blasenkarzinom, Epithelkarzinom, Gliom, Astrocytom, Medulloblastom, Kraniopharyngiom, Ependymom, Pinealom, Hämangioblastom, akustischem Neurom, Oligodendrogliom, Meningiom, Melanom, Neuroblastom, Retinoblastom, Leukämie, Lymphom, multiplem Myelom, Waldenströms Makroglobulinämie und Schwere-Kettenerkrankung handelt.

14. Verwendung nach Anspruch 12, wobei das virale Pathogen der viralen Erkrankungen ausgewählt ist aus der Gruppe, bestehend aus Hepatitis Typ A, Hepatitis Typ B, Hepatitis Typ C, Influenza, Varicella, Adenovirus, Herpes-Simplex Typ I (HSV-I), Herpes Simplex Typ 11 (HSV-II), Rinderpest, Rhinovirus, Echovirus, Rotavirus, respiratorischem Synzytialvirus (RSV), Papillomvirus, Papovavirus, Cytomegalievirus, Echinovirus, Arbovirus, Huntavirus, Coxsackievirus, Mumpsvirus, Masernvirus, Rötelnvirus, Poliovirus, menschliches Immunschwächevirus Typ I (HIV-I) und menschliches Immunschwächevirus Typ II (HIV-II).

15. Verwendung nach Anspruch 12, wobei es sich bei den entzündungsbedingten Erkrankungen um Rheumatoide Arthritis, Asthma, Multiple Sklerose, Typ 1 Diabetes, Lupus Erythematodes, Psoriasis und Inflammatory Bowel Disease handelt.

16. Verwendung nach Anspruch 12, wobei es sich bei den Erkrankungen im Zusammenhang mit Angiogenese um diabetische Retinopathie, Hämangiome, Endometriose und Tumorangiogenese handelt.

17. Verwendung nach Anspruch 12, wobei es sich bei den fibrogenetischen Erkrankungen um Sklerodermie, Polymyositis, systemischer Lupus, Leberzirrhose, Keloidbildung, interstitielle Nephritis und pulmonare Fibrose handelt.

18. Verwendung nach Anspruch 12, wobei es sich bei den Krankheiten, bei denen Proteinfehlfaltung oder Aggregation ein Hauptkausalfaktor ist, um Skrapie, Creutzfeldt-Jakob-Krankheit, Huntington oder Alzheimer handelt.

19. Arzneimittel enthaltend mindestens eine Verbindung der Formel I und/oder ihre pharmazeutisch verwendbaren Salze, Solvate, Tautomere und Stereoisomere, einschließlich deren Mischungen in allen Verhältnissen, und mindestens einen weiteren Arzneimittelwirkstoff.

20. Set (Kit), bestehend aus getrennten Packungen von
(a) einer wirksamen Menge an einer Verbindung der Formel I und/oder ihrer pharmazeutisch verwendbaren Salze, Solvate, Tautomere und Stereoisomere, einschließlich deren Mischungen in allen Verhältnissen,
und
(b) einer wirksamen Menge eines weiteren Arzneimittelswirkstoffs.

## Claims

1. Compounds of the formula I 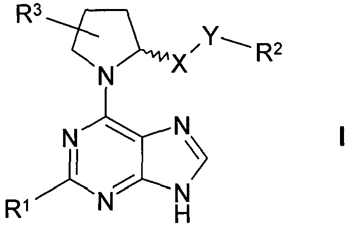 in which
X denotes CH₂ or CO,
Y denotes O, S, NH(CH₂)ₙ or CH₂,
R¹ denotes H,
R² denotes Ar or Het,
R³ denotes H, OH, (CH₂)ₙOH or (CH₂)ₚCH(OH)(CH₂)ₚOH,
A, A' each, independently of one another, denote unbranched or branched alkyl having 1-10 C atoms, in which 1-5 H atoms may be replaced by F, Cl and/or Br,
Alk or cyclic alkyl having 3-7 C atoms,
A and A' together also denote an alkylene chain having 2, 3, 4, 5 or 6 C atoms, in which one or two CH₂ groups may be replaced by O, S, SO, SO₂, NH, NA and/or N-COOA,
Alk denotes alkenyl having 2-6 C atoms,
Ar denotes phenyl, naphthyl or biphenyl, each of which is unsubstituted or mono-, di-, tri-, tetra- or pentasubstituted by A, OA, OH, SH, SA, Hal, NO₂, CN, (CH₂)ₙAr', (CH₂)nCOOH, (CH₂)nCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA', NACONAA', NHCO(CH₂)ₙNH₂ and/or -O-(CH₂)ₒ-Het¹,
Ar' denotes phenyl, naphthyl or biphenyl, each of which is unsubstituted or mono-, di- or trisubstituted by A, OA, OH, SH, SA, Hal, NO₂, CN, (CH₂)ₙphenyl, (CH₂)ₙCOOH, (CH₂)ₙCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA' and/or NACONAA',
Het denotes a mono- or bicyclic saturated, unsaturated or aromatic heterocycle having 1 to 4 N, O and/or S atoms, which may be mono-, di- or trisubstituted by A, OA, OH, SH, SA, Hal, NO₂, CN, (CH₂)ₙAr', (CH₂)ₙCOOH, (CH₂)ₙCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA', NACONAA', SO₂A, =S, =NH, =NA and/or =O (carbonyl oxygen),
Het¹ denotes a monocyclic saturated heterocycle having 1 to 2 N and/or O atoms, which may be mono- or disubstituted by A, OA, OH, Hal and/or =O (carbonyl oxygen),
Hal denotes F, Cl, Br or I,
m denotes 2, 3, 4, 5 or 6,
n denotes 0, 1, 2, 3 or 4,
o denotes 1 or 2,
p denotes 1, 2, 3 or 4,
and pharmaceutically usable salts, solvates, tautomers and stereoisomers thereof, including mixtures thereof in all ratios.

2. Compounds according to Claim 1 of the formula I in which
Y denotes O or NH(CH₂)ₙ,
and pharmaceutically usable salts, solvates, tautomers and stereoisomers thereof, including mixtures thereof in all ratios.

3. Compounds according to Claim 1 or 2 in which
R³ denotes H, OH or (CH₂)ₚCH(OH)(CH₂)ₚOH,
and pharmaceutically usable salts, solvates, tautomers and stereoisomers thereof, including mixtures thereof in all ratios.

4. Compounds according to one or more of Claims 1-3 in which
Ar denotes phenyl which is unsubstituted or mono-, di-, tri-, tetra- or pentasubstituted by A, Hal, OA, OH or CN,
and pharmaceutically usable salts, solvates, tautomers and stereoisomers thereof, including mixtures thereof in all ratios.

5. Compounds according to one or more of Claims 1-4 in which
Het denotes a monocyclic aromatic heterocycle having 1 to 3 N atoms, which may be mono-, di- or trisubstituted by A, OA and/or Hal,
and pharmaceutically usable salts, solvates, tautomers and stereoisomers thereof, including mixtures thereof in all ratios.

6. Compounds according to one or more of Claims 1-5 in which
A denotes unbranched or branched alkyl having 1-6 C atoms, in which 1-5 H atoms may be replaced by F and/or Cl, or Alk,
and pharmaceutically usable salts, solvates, tautomers and stereoisomers thereof, including mixtures thereof in all ratios.

7. Compounds according to one or more of Claims 1-6 in which
X denotes CH₂ or CO,
Y denotes O or NH(CH₂)ₙ,
R¹ denotes H,
R² denotes Ar or Het,
R³ denotes H, OH or (CH₂)ₚCH(OH)(CH₂)ₚOH,
A denotes unbranched or branched alkyl having 1-6 C atoms, in which 1-5 H atoms may be replaced by F and/or Cl, or Alk,
Alk denotes alkenyl having 2-6 C atoms,
Ar denotes phenyl which is unsubstituted or mono-, di-, tri-, tetra- or pentasubstituted by A, Hal, OA, OH or CN,
Het denotes a monocyclic aromatic heterocycle having 1 to 3 N atoms, which may be mono-, di- or trisubstituted by A, OA and/or Hal,
Hal denotes F, Cl, Br or I,
p denotes 1, 2, 3 or 4,
n denotes 0, 1 or 2,
and pharmaceutically usable salts, solvates, tautomers and stereoisomers thereof, including mixtures thereof in all ratios.

8. Compounds according to Claim 1 selected from the group
| Example | Name |
|---|---|
| "A1" | 6-[(R)-2-(5-Fluoro-2-methoxyphenoxymethyl)pyrrolidin-1-yl]-9*H*-purine |
| "A2" | 6-[(R)-2-(4-Chlorophenoxymethyl)pyrrolidin-1-yl]-9*H-*purine |
| "A3" | 6-[(R)-2-(3,4-Difluorophenoxymethyl)pyrrolidin-1-yl]-9*H-*purine |
| "A4" | 6-[(R)-2-(4-Methylphenoxymethyl)pyrrolidin-1-yl]-9*H-*purine |
| "A5" | 6-[(R)-2-(Pyridin-2-yloxymethyl)pyrrolidin-1-yl]-9*H*-purine |
| "A6" | 6-[(R)-2-(6-Methylpyridin-2-yloxymethyl)pyrrolidin-1-yl]-9*H*-purine |
| "A7" | 6-[(R)-2-(2,5-Difluorophenoxymethyl)pyrrolidin-1-yl]-9*H-*purine |
| "A8" | 6-[(R)-2-(3,5-Difluorophenoxymethyl)pyrrolidin-1-yl]-9*H-*purine |
| "A9" | 6-[(R)-2-(2,4-Difluorophenoxymethyl)pyrrolidin-1-yl]-9*H-*purine |
| "A10" | 6-[(R)-2-(2-Fluorophenoxymethyl)pyrrolidin-1-yl]-9*H-*purine |
| "A11" | 6-[(R)-2-(3-Fluorophenoxymethyl)pyrrolidin-1-yl]-9*H-*purine |
| "A12" | 6-[(R)-2-(2,3-Difluorophenoxymethyl)pyrrolidin-1-yl]-9*H-*purine |
| "A13" | 6-[(R)-2-(4-Methoxyphenoxymethyl)pyrrolidin-1-yl]-9*H-*purine |
| "A14" | 6-[(R)-2-(4-Cyanophenoxymethyl)pyrrolidin-1-yl]-9*H-*purine |
| "A15" | 6-[(R)-2-(4-Fluorophenoxymethyl)pyrrolidin-1-yl]-9*H-*purine |
| "A16" | 6-[(R)-2-(2-Methoxyphenoxymethyl)pyrrolidin-1-yl]-9*H-*purine |
| "A17" | 6-[(R)-2-(2-Ethylphenoxymethyl)pyrrolidin-1-yl]-9*H-*purine |
| "A18" | 6-[(R)-2-(2-Methylphenoxymethyl)pyrrolidin-1-yl]-9*H-*purine |
| "A19" | 6-[(R)-2-(Phenoxymethyl)pyrrolidin-1-yl]-9*H*-purine |
| "A20" | 6-[(R)-2-(2-Chlorophenoxymethyl)pyrrolidin-1-yl]-9*H-*purine |
| "A21 | 6-[(R)-2-(2,3-Dichlorophenoxymethyl)pyrrolidin-1-yl]-9*H-*purine |
| "A22" | 6-[(R)-2-(2-Trifluoromethylphenoxymethyl)pyrrolidin-1-yl]-9*H*-purine |
| "A23" | 6-[(R)-2-(2,4-Dichlorophenoxymethyl)pyrrolidin-1-yl]-9*H-*purine |
| "A24" | 6-[(R)-2-(2-Chloro-5-methylphenoxymethyl)pyrrolidin-1-yl]-9*H*-purine |
| "A25" | 6-[(R)-2-(2,6-Dichlorophenoxymethyl)pyrrolidin-1-yl]-9*H-*purine |
| "A26" | 6-[R)-2-(2,5-Dichlorophenoxymethyl)pyrrolidin-1-yl]-9*H-*purine |
| "A27" | 6-[(R)-2-(2-Chloro-4-fluorophenoxymethyl)pyrrolidin-1-yl]-9*H*-purine |
| "A28" | 6-[(R)-2-(2-Chloro-4-methoxyphenoxymethyl)pyrrolidin-1-yl]-9*H*-purine |
| "A29" | 2-Chloro-6-[(R)-2-(2-chlorophenoxymethyl)pyrrolidin-1-yl]-9*H*-purine |
| "A30" | 6-[(R)-2-(4-Chloropyridin-3-yloxymethyl)pyrrolidin-1-yl]-9*H*-purine |
| "A31" | 6-{(R)-2-[(2-Fluorophenylamino)methyl]pyrrolidin-1-yl}-9*H*-purine |
| "A32" | 6-{(R)-2-[(Phenylamino)methyl]pyrrolidin-1-yl}-9*H*-purine |
| "A33" | 6-{(R)-2-[(2-Chlorophenylamino)methyl]pyrrolidin-1-yl}-9*H*-purine |
| "A34" | 6-{(R)-2-[(2-Chlorophenylamino)carbonyl]pyrrolidin-1-yl}-9*H*-purine |
| "A35" | 6-{(R)-2-[(2-Fluorophenylamino)carbonyl]pyrrolidin-1-yl}-9*H*-purine |
| "A36" | 2-Amino-6-[(R)-2-(2-chlorophenoxymethyl)pyrrolidin-1-yl]-9*H*-purine |
| "A37" | 2-Fluoro-6-[(R)-2-(2-chlorophenoxymethyl)pyrrolidin-1-yl]-9*H*-purine |
| "A38" | 2-Chloro-6-[(R)-2-(2-fluorophenoxymethyl)pyrrolidin-1-yl]-9*H*-purine |
| "A40" | 4-[(2S,5R)-5-(2-chlorophenoxymethyl)-1-(9*H*-purin-6-yl)pyrrolidin-2-yl]butane-1,2-diol |
| "A41" | 3-[(2S,5R)-5-(2-chlorophenoxymethyl)-1-(9*H*-purin-6-yl)pyrrolidin-2-yl]propan-1-ol |
| "A42" | 6-{(R)-2-[(2-Fluorobenzylamino)carbonyl]pyrrolidin-1-yl}-9*H*-purine |
| | 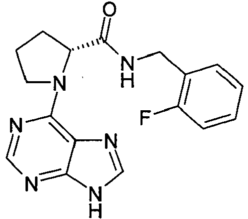 |
| "A43" | 6-{(R)-2-[(4-Fluorobenzylamino)carbonyl]pyrrolidin-1-yl}-9*H*-purine |
| "A44" | 6-[(S)-2-(2-Chlorophenoxymethyl)pyrrolidin-1-yl]-9*H-*purine |
| "A45" | 6-[(S)-2-(Phenoxymethyl)pyrrolidin-1-yl]-9*H*-purine |
| "A46" | 6-[(S)-2-(2-Methylphenoxymethyl)pyrrolidin-1-yl]-9*H-*purine |
| "A47" | 2-Fluoro-6-[(R)-2-(2-fluorophenoxymethyl)pyrrolidin-1-yl]-9*H*-purine |
| "A48" | 2-Chloro-6-[(2R,4S)-2-(2-chlorophenoxymethyl)-4-prop-2-ynyloxypyrrolidin-1-yl]-9*H*-purine |
| "A49" | 6-[(2R,4S)-2-(2-Chlorophenoxymethyl)-4-prop-2-ynyl-oxypyrrolidin-1-yl]-9*H*-purine |
| "A50" | 2-Chloro-6-[(2R,4S)-2-(2-chlorophenoxymethyl)-4-allyl-oxypyrrolidin-1-yl]-9*H*-purine |
| "A51" | 6-[(2R,4S)-2-(2-Chlorophenoxymethyl)-4-allyloxy-pyrrolidin-1-yl]-9*H*-purine |
| "A52" | 6-[(R)-2-(2-Bromophenoxymethyl)pyrrolidin-1-yl]-9*H-*purine |
| "A53" | 2-Chloro-6-[(2R,4S)-2-phenoxymethyl-4-hydroxy-pyrrolidin-1-yl]-9*H*-purine |
| "A54" | 2-Chloro-6-[(2R,4S)-2-(2-fluorophenoxymethyl)-4-hydroxypyrrolidin-1-yl]-9*H*-purine |
| "A55" | 2-Chloro-6-[(2R,4S)-2-(2-fluorophenoxymethyl)-4-prop-2-ynyloxypyrrolidin-1-yl]-9*H*-purine |
| "A56" | 2-Chloro-6-[(2R,4S)-2-phenoxymethyl-4-prop-2-ynyloxy-pyrrolidin-1-yl]-9*H*-purine |
| "A57" | 2-Methyl-6-[(R)-2-(2-fluorophenoxymethyl)pyrrolidin-1-yl]-9*H*-purine |
| "A58" | 2-Methyl-6-[(R)-2-(2-chlorophenoxymethyl)pyrrolidin-1-yl]-9*H*-purine |
| "A59" | 6-[(R)-2-(2-Chlorophenoxymethyl)pyrrolidin-1-yl]-9*H-*purine |
and pharmaceutically usable salts, solvates, tautomers and stereoisomers thereof, including mixtures thereof in all ratios.

9. Process for the preparation of compounds of the formula I according to Claims 1-8 and pharmaceutically usable solvates, salts, tautomers and stereoisomers thereof, **characterised in that**
a compound of the formula II 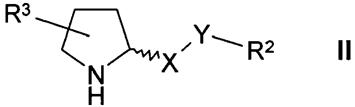 in which
X, Y, R² and R³ have the meanings indicated in Claim 1,
is reacted with
a compound of the formula III 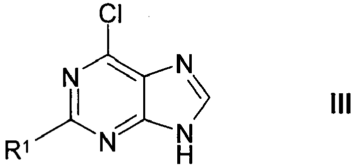
in which R¹ has the meaning indicated in Claim 1,
and/or a base or acid of the formula I is converted into one of its salts.

10. Medicaments comprising at least one compound of the formula I and/or pharmaceutically usable salts, solvates, tautomers and stereoisomers thereof, including mixtures thereof in all ratios, and optionally excipients and/or adjuvants.

11. Use of compounds of the formula I and pharmaceutically usable salts, solvates, tautomers and stereoisomers thereof, including mixtures thereof in all ratios, for the preparation of a medicament for the treatment and/or prophylaxis of diseases in which the inhibition, regulation and/or modulation of HSP90 plays a role.

12. Use according to Claim 11 of compounds of the formula I and pharmaceutically usable salts, solvates, tautomers and stereoisomers thereof, including mixtures thereof in all ratios, for the preparation of a medicament for the treatment or prevention of tumour diseases, viral diseases, for immune suppression in transplants, inflammation-induced diseases, cystic fibrosis, diseases associated with angiogenesis, infectious diseases, autoimmune diseases, ischaemia, fibrogenetic diseases,
for the promotion of nerve regeneration,
for inhibiting the growth of cancer, tumour cells and tumour metastases,
for the protection of normal cells against toxicity caused by chemotherapy,
for the treatment of diseases in which incorrect protein folding or aggregation is a principal causal factor.

13. Use according to Claim 12, where the tumour diseases are fibro-sarcoma, myxosarcoma, liposarcoma, chondrosarcoma, osteogenic sarcoma, chordoma, angiosarcoma, endotheliosarcoma, lymphangio-sarcoma, lymphangioendotheliosarcoma, synovioma, mesothelioma, Ewing's tumour, leiosarcoma, rhabdomyosarcoma, colon carcinoma, pancreatic cancer, breast cancer, ovarian cancer, prostate cancer, squamous cell carcinoma, basal cell carcinoma, adenocarcinoma, syringocarcinoma, sebaceous gland carcinoma, papillary carcinoma, papillary adenocarcinomas, cystadenocarcinomas, bone marrow carcinoma, bronchogenic carcinoma, renal cell carcinoma, hepatoma, bile duct carcinoma, choriocarcinoma, seminoma, embryonic carcinoma, Wilm's tumour, cervical cancer, testicular tumour, lung carcinoma, small-cell lung carcinoma, bladder carcinoma, epithelial carcinoma, glioma, astrocytoma, medulloblastoma, craniopharyngioma, ependymoma, pinealoma, haemangioblastoma, acoustic neuroma, oligodendroglioma, meningioma, melanoma, neuroblastoma, retino-blastoma, leukaemia, lymphoma, multiple myeloma, Waldenström's macroglobulinaemia and heavy chain disease.

14. Use according to Claim 12, where the viral pathogen of the viral diseases is selected from the group consisting of hepatitis type A, hepatitis type B, hepatitis type C, influenza, varicella, adenovirus, herpes simplex type I (HSV-I), herpes simplex type II (HSV-II), cattle plague, rhinovirus, echovirus, rotavirus, respiratory syncytial virus (RSV), papillomavirus, papovavirus, cytomegalovirus, echinovirus, arbovirus, huntavirus, Coxsackie virus, mumps virus, measles virus, rubella virus, polio virus, human immunodeficiency virus type I (HIV-I) and human immunodeficiency virus type II (HIV-II).

15. Use according to Claim 12, where the inflammation-induced diseases are rheumatoid arthritis, asthma, multiple sclerosis, type 1 diabetes, lupus erythematosus, psoriasis and inflammatory bowel disease.

16. Use according to Claim 12, where the diseases associated with angiogenesis are diabetic retinopathy, haemangiomas, endometriosis and tumour angiogenesis.

17. Use according to Claim 12, where the fibrogenetic diseases are scleroderma, polymyositis, systemic lupus, cirrhosis of the liver, keloid formation, interstitial nephritis and pulmonary fibrosis.

18. Use according to Claim 12, where the diseases in which incorrect protein folding or aggregation is a principal causal factor are scrapie, Creutzfeldt-Jakob disease, Huntington's or Alzheimer's.

19. Medicaments comprising at least one compound of the formula I and/or pharmaceutically usable salts, solvates, tautomers and stereoisomers thereof, including mixtures thereof in all ratios, and at least one further medicament active ingredient.

20. Set (kit) consisting of separate packs of
(a) an effective amount of a compound of the formula I and/or pharmaceutically usable salts, solvates, tautomers and stereoisomers thereof, including mixtures thereof in all ratios,
and
(b) an effective amount of a further medicament active ingredient.

## Revendications

1. Composés de formule I 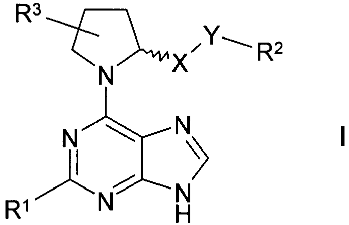 dans laquelle
X désigne CH₂ ou CO,
Y désigne O, S, NH(CH₂)ₙ ou CH₂,
R¹ désigne H,
R² désigne Ar ou Hét,
R³ désigne H, OH, (CH₂)ₙOH ou (CH₂)ₚCH(OH)(CH₂)ₚOH,
A, A' désignent chacun, indépendamment l'un de l'autre, alkyle non ramifié ou ramifié ayant 1-10 atomes de C, où 1-5 atomes de H peuvent être remplacés par F, Cl et/ou Br,
Alk ou alkyle cyclique ayant 3-7 atomes de C,
A et A' désignent aussi ensemble une chaîne alkylène ayant 2, 3, 4, 5 ou 6 atomes de C, où un ou deux groupements CH₂ peuvent être remplacés par O, S, SO, SO₂, NH, NA et/ou N-COOA,
Alk désigne alcényle ayant 2-6 atomes de C,
Ar désigne phényle, naphtyle ou biphényle, chacun d'entre eux étant non substitué ou mono-, di-, tri-, tétra- ou pentasubstitué par A, OA, OH, SH, SA, Hal, NO₂, CN, (CH₂)ₙAr', (CH₂)ₙCOOH, (CH₂)ₙCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA', NACONAA', NHCO(CH₂)ₙNH₂ et/ou -O-(CH₂)ₒ-Hét¹,
Ar' désigne phényle, naphtyle ou biphényle, chacun d'entre eux étant non substitué ou mono-, di- ou trisubstitué par A, OA, OH, SH, SA, Hal, NO₂, CN, (CH₂)ₙphényle, (CH₂)ₙCOOH, (CH₂)ₙCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA' et/ou NACONAA',
Hét désigne un hétérocycle mono- ou bicyclique saturé, insaturé ou aromatique ayant de 1 à 4 atomes de N, O et/ou S, qui peut être mono-, di- ou trisubstitué par A, OA, OH, SH, SA, Hal, NO₂, CN, (CH₂)ₙAr', (CH₂)ₙCOOH, (CH₂)ₙCOOA, CHO, COA, SO₂A, CONH₂, SO₂NH₂, CONHA, CONAA', SO₂NHA, SO₂NAA', NH₂, NHA, NAA', OCONH₂, OCONHA, OCONAA', NHCOA, NHCOOA, NACOOA, NHSO₂OA, NASO₂OA, NHCONH₂, NACONH₂, NHCONHA, NACONHA, NHCONAA', NACONAA', SO₂A, =S, =NH, =NA et/ou =O (oxygène du carbonyle),
Hét¹ désigne un hétérocycle monocyclique saturé ayant de 1 à 2 atomes de N et/ou O, qui peut être mono- ou disubstitué par A, OA, OH, Hal et/ou =O (oxygène du carbonyle),
Hal désigne F, Cl, Br ou I,
m désigne 2, 3, 4, 5 ou 6,
n désigne 0, 1, 2, 3 ou 4,
o désigne 1 ou 2,
p désigne 1, 2, 3 ou 4,
et les sels, solvats, tautomères et stéréoisomères pharmaceutiquement utilisables de ceux-ci, y compris des mélanges de ceux-ci en toutes proportions.

2. Composés selon la revendication 1 de formule I, dans lesquels
Y désigne O ou NH(CH₂)ₙ,
et les sels, solvats, tautomères et stéréoisomères pharmaceutiquement utilisables de ceux-ci, y compris des mélanges de ceux-ci en toutes proportions.

3. Composés selon la revendication 1 ou 2, dans lesquels
R³ désigne H, OH ou (CH₂)ₚCH(OH)(CH₂)ₚOH,
et les sels, solvats, tautomères et stéréoisomères pharmaceutiquement utilisables de ceux-ci, y compris des mélanges de ceux-ci en toutes proportions.

4. Composés selon l'une ou plusieurs parmi les revendications 1-3, dans lesquels
Ar désigne phényle qui est non substitué ou mono-, di-, tri-, tétra- ou pentasubstitué par A, Hal, OA, OH ou CN,
et les sels, solvats, tautomères et stéréoisomères pharmaceutiquement utilisables de ceux-ci, y compris des mélanges de ceux-ci en toutes proportions.

5. Composés selon l'une ou plusieurs parmi les revendications 1-4, dans lesquels
Hét désigne un hétérocycle monocyclique aromatique ayant de 1 à 3 atomes de N, qui peut être mono-, di- ou trisubstitué par A, OA et/ou Hal,
et les sels, solvats, tautomères et stéréoisomères pharmaceutiquement utilisables de ceux-ci, y compris des mélanges de ceux-ci en toutes proportions.

6. Composés selon l'une ou plusieurs parmi les revendications 1-5, dans lesquels
A désigne alkyle non ramifié ou ramifié ayant 1-6 atomes de C, où 1-5 atomes de H peuvent être remplacés par F et/ou Cl, ou Alk,
et les sels, solvats, tautomères et stéréoisomères pharmaceutiquement utilisables de ceux-ci, y compris des mélanges de ceux-ci en toutes proportions.

7. Composés selon l'une ou plusieurs parmi les revendications 1-6, dans lesquels
X désigne CH₂ ou CO,
Y désigne O ou NH(CH₂)ₙ,
R¹ désigne H,
R² désigne Ar ou Hét,
R³ désigne H, OH ou (CH₂)pCH(OH)(CH₂)pOH,
A désigne alkyle non ramifié ou ramifié ayant 1-6 atomes de C, où 1-5 atomes de H peuvent être remplacés par F et/ou Cl, ou Alk,
Alk désigne alcényle ayant 2-6 atomes de C,
Ar désigne phényle qui est non substitué ou mono-, di-, tri-, tétra- ou pentasubstitué par A, Hal, OA, OH ou CN,
Hét désigne un hétérocycle monocyclique aromatique ayant de 1 à 3 atomes de N, qui peut être mono-, di- ou trisubstitué par A, OA et/ou Hal,
Hal désigne F, Cl, Br ou I,
p désigne 1, 2, 3 ou 4,
n désigne 0, 1 ou 2,
et les sels, solvats, tautomères et stéréoisomères pharmaceutiquement utilisables de ceux-ci, y compris des mélanges de ceux-ci en toutes proportions.

8. Composés selon la revendication 1, choisis dans le groupe constitué par
| Exemple | Nom |
|---|---|
| "A1" | 6-[(R)-2-(5-Fluoro-2-méthoxyphénoxyméthyl)pyrrolidin-1-yl]-9*H*-purine |
| "A2" | 6-[(R)-2-(4-Chlorophénoxyméthyl)pyrrolidin-1-yl]-9*H-*purine |
| "A3" | 6-[(R)-2-(3,4-Difluorophénoxyméthyl)pyrrolidin-1-yl]-9*H-*purine |
| "A4" | 6-[(R)-2-(4-Méthylphénoxyméthyl)pyrrolidin-1-yl]-9*H-*purine |
| "A5" | 6-[(R)-2-(Pyridin-2-yloxyméthyl)pyrrolidin-1-yl]-9*H*-purine |
| "A6" | 6-[(R)-2-(6-Méthylpyridin-2-yloxyméthyl)pyrrolidin-1-yl]-9*H*-purine |
| "A7" | 6-[(R)-2-(2,5-Difluorophénoxyméthyl)pyrrolidin-1-yl]-9*H-*purine |
| "A8" | 6-[(R)-2-(3,5-Difluorophénoxyméthyl)pyrrolidin-1-yl]-9*H-*purine |
| "A9" | 6-[(R)-2-(2,4-Difluorophénoxyméthyl)pyrrolidin-1-yl]-9*H-*purine |
| "A10" | 6-[(R)-2-(2-Fluorophénoxyméthyl)pyrrolidin-1-yl]-9*H-*purine |
| "A11" | 6-[(R)-2-(3-Fluorophénoxyméthyl)pyrrolidin-1-yl]-9*H-*purine |
| "A12" | 6-[(R)-2-(2,3-Difluorophénoxyméthyl)pyrrolidin-1-yl]-9*H-*purine |
| "A13" | 6-[(R)-2-(4-Méthoxyphénoxyméthyl)pyrrolidin-1-yl]-9*H-*purine |
| "A14" | 6-[(R)-2-(4-Cyanophénoxyméthyl)pyrrolidin-1-yl]-9*H-*purine |
| "A15" | 6-[(R)-2-(4-Fluorophénoxyméthyl)pyrrolidin-1-yl]-9*H-*purine |
| "A16" | 6-[(R)-2-(2-Méthoxyphénoxyméthyl)pyrrolidin-1-yl]-9*H-*purine |
| "A17" | 6-[(R)-2-(2-Éthylphénoxyméthyl)pyrrolidin-1-yl]-9*H-*purine |
| "A18" | 6-[(R)-2-(2-Méthylphénoxyméthyl)pyrrolidin-1-yl]-9*H-*purine |
| "A19" | 6-[(R)-2-(Phénoxyméthyl)pyrrolidin-1-yl]-9*H*-purine |
| "A20" | 6-[(R)-2-(2-Chlorophénoxyméthyl)pyrrolidin-1-yl]-9*H-*purine |
| "A21 | 6-[(R)-2-(2,3-Dichlorophénoxyméthyl)pyrrolidin-1-yl]-9*H-*purine |
| "A22" | 6-[(R)-2-(2-Trifluorométhylphénoxyméthyl)pyrrolidin-1-yl]-9*H*-purine |
| "A23" | 6-[(R)-2-(2,4-Dichlorophénoxyméthyl)pyrrolidin-1-yl]-9*H-*purine |
| "A24" | 6-[(R)-2-(2-Chloro-5-méthylphénoxyméthyl)pyrrolidin-1-yl]-9*H*-purine |
| "A25" | 6-[(R)-2-(2,6-Dichlorophénoxyméthyl)pyrrolidin-1-yl]-9*H-*purine |
| "A26" | 6-[(R)-2-(2, 5-Dichlorophénoxyméthyl)pyrrolidin-1-yl]-9*H-*purine |
| "A27" | 6-[(R)-2-(2-Chloro-4-fluorophénoxyméthyl)pyrrolidin-1-yl]-9*H*-purine |
| "A28" | 6-[(R)-2-(2-Chloro-4-méthoxyphénoxyméthyl)pyrrolidin-1-yl]-9*H*-purine |
| "A29" | 2-Chloro-6-[(R)-2-(2-chlorophénoxyméthyl)pyrrolidin-1-yl]-9*H*-purine |
| "A30" | 6-[(R)-2-(4-Chloropyridin-3-yloxyméthyl)pyrrolidin-1-yl]-9*H*-purine |
| "A31" | 6-{(R)-2-[(2-Fluorophénylamino)méthyl]pyrrolid in-1-yl}-9*H*-purine |
| "A32" | 6-{(R)-2-[(Phénylamino)méthyl]pyrrolidin-1-yl}-9*H*-purine |
| "A33" | 6-{(R)-2-[(2-Chlorophénylamino)méthyl]pyrrolidin-1-yl}-9*H*-purine |
| "A34" | 6-{(R)-2-[(2-Chlorophénylamino)carbonyl]pyrrolidin-1-yl}-9*H*-purine |
| "A35" | 6-{(R)-2-[(2-Fluorophénylamino)carbonyl]pyrrolidin-1-yl}-9*H*-purine |
| "A36" | 2-Amino-6-[(R)-2-(2-chlorophénoxyméthyl)pyrrolidin-1-yl]-9*H*-purine |
| "A37" | 2-Fluoro-6-[(R)-2-(2-chlorophénoxyméthyl)pyrrolidin-1-yl]-9*H*-purine |
| "A38" | 2-Chloro-6-[(R)-2-(2-fluorophénoxyméthyl)pyrrolidin-1-yl]-9*H*-purine |
| "A40" | 4-[(2S,5R)-5-(2-chlorophénoxyméthyl)-1-(9*H*-purin-6-yl)pyrrolidin-2-yl]butane-1,2-diol |
| "A41" | 3-[(2S,5R)-5-(2-chlorophénoxyméthyl)-1-(9*H*-purin-6-yl)pyrrolidin-2-yl]propan-1-ol |
| "A42" | 6-{(R)-2-[(2-Fluorobenzylamino)carbonyl]pyrrolidin-1 -yl}-9*H*-purine |
| | 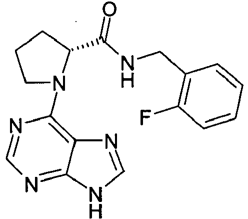 |
| "A43" | 6-{(R)-2-[(4-Fluorobenzylamino)carbonyl]pyrrolidin-1-yl}-9*H*-purine |
| "A44" | 6-[(S)-2-(2-Chlorophénoxyméthyl)pyrrolidin-1-yl]-9*H-*purine |
| "A45" | 6-[(S)-2-(Phénoxyméthyl)pyrrolidin-1-yl]-9*H*-purine |
| "A46" | 6-[(S)-2-(2-Méthylphénoxyméthyl)pyrrolidin-1-yl]-9*H-*purine |
| "A47" | 2-Fluoro-6-[(R)-2-(2-fluorophénoxyméthyl)pyrrolidin-1-yl]-9*H*-purine |
| "A48" | 2-Chloro-6-[(2R,4S)-2-(2-chlorophénoxyméthyl)-4-prop-2-ynyloxypyrrolidin-1-yl]-9*H*-purine |
| "A49" | 6-[(2R,4S)-2-(2-Chlorophénoxyméthyl)-4-prop-2-ynyloxypyrrolidin-1-yl]-9*H*-purine |
| "A50" | 2-Chloro-6-[(2R,4S)-2-(2-chlorophénoxyméthyl)-4-allyloxypyrrolidin-1-yl]-9*H*-purine |
| "A51 | 6-[(2R,4S)-2-(2-Chlorophénoxyméthyl)-4-allyloxypyrrolidin-1-yl]-9*H*-purine |
| "A52" | 6-[(R)-2-(2-Bromophénoxyméthyl)pyrrolidin-1-yl]-9*H-*purine |
| "A53" | 2-Chloro-6-[(2R,4S)-2-phénoxyméthyl-4-hydroxypyrrolidin-1-yl]-9*H*-purine |
| "A54" | 2-Chloro-6-[(2R,4S)-2-(2-fluorophénoxyméthyl)-4-hydroxypyrrolidin-1-yl]-9*H*-purine |
| "A55" | 2-Chloro-6-[(2R,4S)-2-(2-fluorophénoxyméthyl)-4-prop-2-ynyloxypyrrolidin-1-yl]-9*H*-purine |
| "A56" | 2-Chloro-6-[(2R,4S)-2-phénoxyméthyl-4-prop-2-ynyloxypyrrolidin-1-yl]-9*H*-purine |
| "A57" | 2-Méthyl-6-[(R)-2-(2-fluorophénoxyméthyl)pyrrolidin-1-yl]-9*H*-purine |
| "A58" | 2-Méthyl-6-[(R)-2-(2-chlorophénoxyméthyl)pyrrolidin-1-yl]-9*H*-purine |
| "A59" | 6-[(R)-2-(2-Chlorophénoxyméthyl)pyrrolidin-1-yl]-9*H-*purine |
et les sels, solvats, tautomères et stéréoisomères pharmaceutiquement utilisables de ceux-ci, y compris des mélanges de ceux-ci en toutes proportions.

9. Procédé de préparation de composés de formule I selon les revendications 1-8 et de solvats, sels, tautomères et stéréoisomères pharmaceutiquement utilisables de ceux-ci, **caractérisé en ce qu'**un composé de formule II 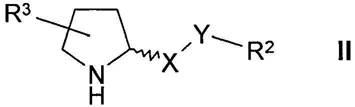
dans laquelle X, Y, R² et R³ revêtent les significations indiquées selon la revendication 1,
est réagi avec un composé de formule III 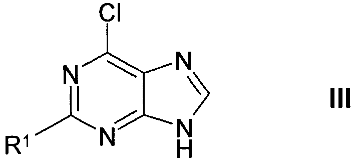
dans laquelle R¹ revêt la signification indiquée selon la revendication 1,
et/ou une base ou un acide de formule I est converti(e) en l'un de ses sels.

10. Médicaments comprenant au moins un composé de formule I et/ou des sels, solvats, tautomères et stéréoisomères pharmaceutiquement utilisables de celui-ci, y compris des mélanges de ceux-ci en toutes proportions, et éventuellement des excipients et/ou des adjuvants.

11. Utilisation de composés de formule I est de sels, solvats, tautomères et stéréoisomères pharmaceutiquement utilisables de ceux-ci, y compris des mélanges de ceux-ci en toutes proportions, pour la préparation d'un médicament destiné au traitement et/ou à la prophylaxie de maladies dans lesquelles l'inhibition, la régulation et/ou la modulation de HSP90 joue un rôle.

12. Utilisation selon la revendication 11 de composés de formule I est de sels, solvats, tautomères et stéréoisomères pharmaceutiquement utilisables de ceux-ci, y compris des mélanges de ceux-ci en toutes proportions, pour la préparation d'un médicament destiné au traitement ou à la prévention de maladies tumorales, de maladies virales, pour la suppression immunitaire dans le cas des greffes, de maladies induites par des inflammations, de la fibrose kystique, de maladies associées à l'angiogenèse, de maladies infectieuses, de maladies auto-immunes, de l'ischémie, de maladies fibrosantes,
pour la promotion de la régénération nerveuse,
pour l'inhibition de la croissance de cancers, de cellules tumorales et de métastases tumorales,
pour la protection de cellules normales contre une toxicité provoquée par une chimiothérapie,
pour le traitement de maladies dans lesquelles un repliement protéique incorrect ou une agrégation constitue un facteur causal principal.

13. Utilisation selon la revendication 12, dans laquelle les maladies tumorales sont le fibrosarcome, le myxosarcome, le liposarcome, le chondrosarcome, le sarcome ostéogénique, le chordome, l'angiosarcome, l'endothéliosarcome, le lymphangiosarcome, le lymphangioendo-théliosarcome, le synoviome, le mésothéliome, le sarcome d'Ewing, le léiomyosarcome, le rhabdomyosarcome, le carcinome du côlon, le cancer du pancréas, le cancer du sein, le cancer de l'ovaire, le cancer de la prostate, le carcinome des cellules squameuses, l'épithélioma basocellulaire, l'adénocarcinome, l'hidradénome, le carcinome des glandes sébacées, le carcinome papillaire, les adénocarcinomes papillaires, les cystadénocarcinomes, le carcinome de la moelle osseuse, le carcinome bronchogénique, le carcinome des cellules rénales, l'hépatome, le carcinome du canal biliaire, le chorio-épithé-liome, le séminome, le carcinome embryonnaire, le néphroblastome, le cancer du col de l'utérus, les tumeurs testiculaires, le carcinome du poumon, le carcinome du poumon à petites cellules, le carcinome de la vessie, le carcinome épithélial, le gliome, l'astrocytome, le médullo-blastome, le craniopharyngiome, l'épendymome, le pinéalome, l'hémangioblastome, le neurinome de l'acoustique, l'oligodendro-gliome, le méningiome, le mélanome, le neuroblastome, le rétino-blastome, la leucémie, le lymphome, le myélome multiple, la macroglobulinémie de Waldenström et la maladie des chaînes lourdes.

14. Utilisation selon la revendication 12, dans laquelle le pathogène viral des maladies virales est choisi dans le groupe constitué par l'hépatite de type A, l'hépatite de type B, l'hépatite de type C, l'influenzavirus, le virus de la varicelle, l'adénovirus, l'herpès simplex de type I (HSV-I), l'herpès simplex de type II (HSV-II), le virus de la peste bovine, le rhinovirus, l'échovirus, le rotavirus, le virus respiratoire syncytial (RSV), le papillomavirus, le papovavirus, le cytomégalovirus, l'échino-virus, l'arbovirus, le hantavirus, le Coxsackie virus, le virus des oreil-lons, le virus de la rougeole, le virus de la rubéole, le poliovirus, le virus d'immunodéficience humaine de type I (VIH-I) et le virus d'immunodéficience humaine de type II (VIH-II).

15. Utilisation selon la revendication 12, dans laquelle les maladies induites par des inflammations sont la polyarthrite rhumatoïde, l'asthme, la sclérose en plaques, le diabète de type 1, le lupus érythémateux, le psoriasis et l'affection abdominale inflammatoire.

16. Utilisation selon la revendication 12, dans laquelle les maladies associées à l'angiogenèse sont la rétinopathie diabétique, les hémangiomes, l'endométriose et l'angiogenèse tumorale.

17. Utilisation selon la revendication 12, dans laquelle les maladies fibrosantes sont la sclérodermie, la polymyosite, le lupus disséminé, la cirrhose du foie, la formation de kéloïdes, la néphrite interstitielle et la fibrose pulmonaire.

18. Utilisation selon la revendication 12, dans laquelle les maladies dans lesquelles un repliement protéique incorrect ou une agrégation constitue un facteur causal principal sont la tremblante du mouton, la maladie de Creutzfeldt-Jakob, la maladie de Huntington ou la maladie d'Alzheimer.

19. Médicaments comprenant au moins un composé de formule I et/ou des sels, solvats, tautomères et stéréoisomères pharmaceutiquement utilisables de celui-ci, y compris des mélanges de ceux-ci en toutes proportions, et au moins un autre ingrédient actif médicamenteux.

20. Ensemble (kit) constitué de conditionnements séparés
(a) d'une quantité efficace d'un composé de formule I et/ou de sels, solvats, tautomères et stéréoisomères pharmaceutiquement utilisables de celui-ci, y compris des mélanges de ceux-ci en toutes proportions,
et
(b) d'une quantité efficace d'un autre ingrédient actif médicamenteux.
